(19) Europäisches Patentamt
European Patent Office
Office européen des brevets

(11) Veröffentlichungsnummer: **0 041 233**
**B1**

(12) # EUROPÄISCHE PATENTSCHRIFT

(45) Veröffentlichungstag der Patentschrift: **15.02.84**

(51) Int. Cl.³: **C 07 C 93/06**, C 07 C 149/32, C 07 D 303/24, C 07 C 43/20, C 07 C 49/84, A 61 K 31/135

(21) Anmeldenummer: **81104078.1**

(22) Anmeldetag: **27.05.81**

(54) **Substituierte Phenoxy-aminopropanol-Derivate, Zwischenprodukte und Verfahren zu ihrer Herstellung sowie diese enthaltende Arzneimittel.**

(30) Priorität: **02.06.80 GB 8017983**
**17.03.81 GB 8108345**

(43) Veröffentlichungstag der Anmeldung: **09.12.81 Patentblatt 81/49**

(45) Bekanntmachung des Hinweises auf die Patenterteilung: **15.02.84 Patentblatt 84/7**

(84) Benannte Vertragsstaaten: **AT BE CH DE FR GB IT LI LU NL SE**

(56) Entgegenhaltungen:
**DD - A - 8 731**
**DE - A - 2 316 727**
**DE - A - 2 400 693**
**DE - B - 2 020 864**

**Die Akte enthält technische Angaben, die nach dem Eingang der Anmeldung eingereicht wurden und die nicht in dieser Patentschrift enthalten sind.**

(73) Patentinhaber: **F. HOFFMANN-LA ROCHE & CO. Aktiengesellschaft, CH-4002 Basel (CH)**

(72) Erfinder: **Machin, Peter James, 8, The Elms Tooting Bec Road, London (GB)**

(74) Vertreter: **Lederer, Franz, Dr. et al, Patentanwälte Dr. Franz Lederer Reiner F. Meyer Lucile-Grahn-Strasse 22, D-8000 München 80 (DE)**

**EP 0 041 233 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

# Substituierte Phenoxy-aminopropanol-Derivate, Zwischenprodukte und Verfahren zu ihrer Herstellung, sowie diese enthaltende Arzneimittel

Die vorliegende Erfindung betrifft substituierte Phenoxy-aminopropanol-Derivate. Im speziellen betrifft sie substituierte Phenoxy-aminopropanol-Derivate, ein Verfahren zu ihrer Herstellung und diese enthaltende pharmazeutische Präparate. Die Erfindung betrifft ebenfalls die Verwendung dieser Derivate.

Die erfindungsgemässen substituierten Phenoxy-amino-propanol-Derivate sind Verbindungen der allgemeinen Formel:

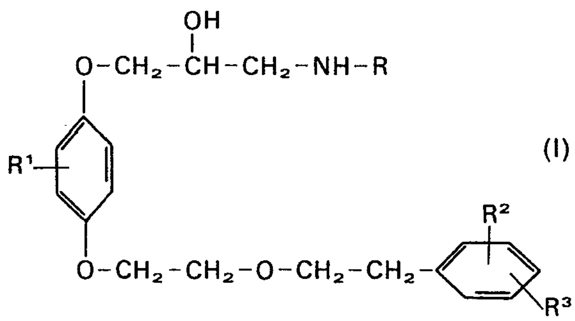

(I)

worin R eine verzweigte, 3 bis 4 Kohlenstoffatome enthaltende Alkylgruppe, $R^1$ Wasserstoff, Halogen oder niederes Alkyl und $R^2$ und $R^3$ je Wasserstoff, Halogen, niederes Alkyl, niederes Alkoxy oder niederes Alkylthio bedeuten, und pharmazeutisch annehmbare Säureadditionssalze davon.

Aus den deutschen Offenlegungsschriften Nrn. 2316727 und 2400693 sowie der deutschen Auslegeschrift Nr. 2020864 sind Phenoxy-amino-propanol-Derivate bekannt, welche in para-Stellung durch eine Alkoxyalkoxy- oder Alkylthioalkoxygruppe substituiert sein können. Diese substituierten Derivate besitzen kardioselektive, β-adrenergische Blockereigenschaften und können demnach bei der Bekämpfung bzw. Verhütung von Herzkrankheiten, wie beispielsweise Angina pectoris oder Herzrythmusstörungen oder auch als antihypertensive Wirkstoffe verwendet werden.

Der in dieser Beschreibung verwendete Ausdruck «niederes Alkyl» bezeichnet geradkettige oder verzweigte Alkylgruppen, welche vorzugsweise 1 bis 4 Kohlenstoffatome enthalten (z.B. Methyl, Äthyl, Propyl, Isopropyl, Butyl, Isobutyl und t-Butyl). Die 3 bis 4 Kohlenstoffatome enthaltende verzweigte Alkylgruppe R ist Isopropyl, Isobutyl, s-Butyl oder t-butyl. Der Ausdruck «niederes Alkoxy» bezeichnet geradkettige oder verzweigte Alkoxygruppen, welche vorzugsweise 1 bis 4 Kohlenstoffatome enthalten (z.B. Methoxy, Äthoxy, Propoxy, Isopropoxy, Butoxy und dergleichen). Der Ausdruck «niederes Alkylthio» bezeichnet Alkylthiogruppen, welche vorzugsweise 1 bis 4 Kohlenstoffatome enthalten (z.B. Methylthio, Äthylthio usw.). Der Ausdruck «Halogen» bedeutet Fluor, Chlor, Brom und Jod.

Eine spezielle Klasse von Verbindungen der allgemeinen Formel I umfasst solche, worin $R^1$ in ortho-Stellung zur 3-Alkylamino-2-hydroxypropoxy-gruppe steht, $R^3$ Wasserstoff bedeutet und R und $R^2$ obige Bedeutung besitzen.

Eine bevorzugte Klasse von Verbindungen der allgemeinen Formel I umfasst solche, worin R Isopropyl oder t-Butyl bedeutet.

Besonders bevorzugte Verbindungen der allgemeinen Formel I sind:

1-/4-[2-(4-Methylphenäthyloxy)äthoxy]phenoxy/-3-isopropylamino-2-propanol,

1-/4-[2-(4-Methylthiophenäthyloxy)äthoxy]-phenoxy/-3-isopropylamino-2-propanol,

1-t-Butylamino-3-[4-(2-phenäthyloxyäthoxy)phenoxy]-2-propanol,

1-/2-Chlor-4-[2-(phenäthyloxy)äthoxy]phenoxy/-3-isopropylamino-2-propanol,

1-/2-Methyl-4-[2-(phenäthyloxy)äthoxy]-phenoxy/-3-isopropylamino-2-propanol,

1-/4-[2-(4-Methoxyphenäthyloxy)äthoxy]-phenoxy/-3-isopropylamino-2-propanol,

1-/4-[2-(4-Chlorphenäthyloxy)äthoxy]phenoxy/-3-isopropylamino-2-propanol,

1-/2-Fluor-4-[2-(4-fluorphenäthyloxy)äthoxy]phenoxy/-3-isopropylamino-2-propanol,

1-/3-Fluor-4-[2-(4-fluorphenäthyloxy)äthoxy]phenoxy/-3-isopropylamino-2-propanol, und

1-/4-[2-(2,4-Difluorphenäthyloxy)äthoxy]-phenoxy/-3-isopropylamino-2-propanol.

Ganz besonders bevorzugte Verbindungen der allgemeinen Formel I sind:

1-Isopropylamino-3-[4-(2-phenäthyloxyäthoxy)phenoxy]-2-propanol, und

1-/4-[2-(4-Fluorphenäthyloxy)äthoxy]phenoxy/-3-isopropylamino-2-propanol.

Die substituierten Phenoxy-aminopropanol-Derivate der allgemeinen Formel I und ihre pharmazeutisch annehmbaren Säureadditionssalze können erfindungsgemäss dadurch hergestellt werden, dass man:

a) ein Epoxid der allgemeinen Formel:

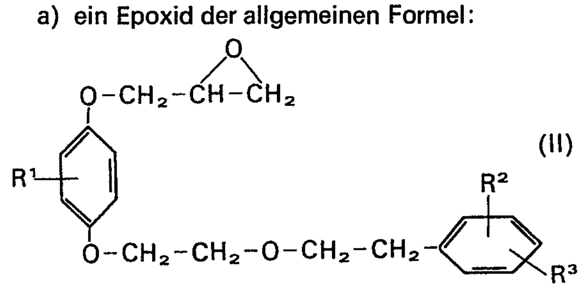

(II)

worin $R^1$, $R^2$ und $R^3$ obige Bedeutung besitzen, mit einem Amin der allgemeinen Formel:

$$H_2N-R \quad (III)$$

worin R obige Bedeutung besitzt, umsetzt, oder

b) ein Alkalimetall-Derivat eines Phenols der allgemeinen Formel:

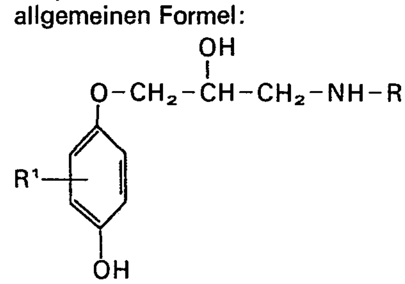

(IV)

worin R und $R^1$ obige Bedeutung besitzen, mit einer Verbindung der allgemeinen Formel:

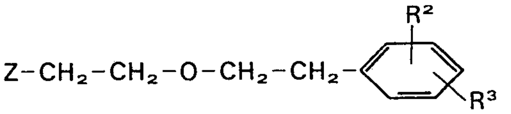

(V)

worin $R^2$ und $R^3$ obige Bedeutung besitzen und Z niederes Alkylsulphonyloxy oder Arylsulphonyloxy bedeutet, umsetzt, und

c) erwünschtenfalls ein erhaltenes Racemat in die optischen Antipoden spaltet, und/oder

d) erwünschtenfalls eine erhaltene Verbindung der allgemeinen Formel I in ein pharmazeutisch annehmbares Säure-additionssalz überführt.

Die Reaktion eines Epoxides der Formel II mit einem Amin der Formel III (das eine bekannte Verbindung ist), gemäss Verfahrensvariante a), kann in an sich bekannter Weise durchgeführt werden. Die Reaktion kann in Gegenwart oder in Abwesenheit eines inerten organischen Lösungsmittels durchgeführt werden. Verwendet man ein inertes organisches Lösungsmittel, so kommen beispielsweise niedere Alkohole, wie Methanol, Äthanol oder dergleichen, in Frage. Andererseits kann auch überschüssiges Amin der Formel III als Lösungsmittel dienen. Vorteilhafterweise arbeitet man in einem Temperaturbereich von etwa 0°C bis etwa Raumtemperatur, vorzugsweise bei Raumtemperatur, und bei Atmosphärendruck.

Die Reaktion eines Alkalimetall-Derivates eines Phenols der Formel IV mit einer Verbindung der Formel V, gemäss Verfahrensvariante b), wird zweckmässigerweise in einem inerten organischen Lösungsmittel durchgeführt. Geeignete inerte organische Lösungsmittel sind beispielsweise Dimethylformamid, Dioxan, Dimethoxyäthan und Tetrahydrofuran, wobei Dimethylformamid bevorzugt wird. Das Alkalimetall-Derivat eines Phenols der Formel IV wird vorzugsweise in situ aus einem entsprechenden Phenol und einem Alkalimetall, einem Alkalimetallhydrid oder einem Alkalimetallamid, vorzugsweise einem Alkalimetallhydrid, insbesondere Natriumhydrid, hergestellt. Die Reaktion wird vorteilhafterweise bei erhöhter Temperatur, vorzugsweise bei etwa 60°C, durchgeführt. Die mit Z bezeichnete Gruppe in einer Verbindung der allgemeinen Formel V ist vorzugsweise eine niedere Alkylsulphonyloxygruppe (z.B. Methansulphonyloxy), kann aber auch eine Arylsulphonyloxygruppe sein (z.B. Benzolsulphonyloxy oder p-Toluolsulphonyloxy).

Es sei besonders hervorgehoben, dass die Verbindungen der allgemeinen Formel I ein asymmetrisch substituiertes Kohlenstoffatom besitzen und somit in racemischen oder optischen aktiven Formen auftreten können. Die vorliegende Erfindung umfasst sowohl die Racemate als auch die optisch aktiven Formen. Gemäss Verfahrensvariante c) kann ein erhaltenes Racemat erwünschtenfalls nach an sich bekannten Methoden in die optischen Antipoden gespalten werden; beispielsweise durch fraktionierte Kristallisation von Salzen, welche mit optisch aktiven Säuren erhalten wurden. Es sei noch erwähnt, dass man das als Ausgangsmaterial verwendete Phenol der Formel IV auch in optisch aktiver Form einsetzen kann, und somit eine optisch aktive Verbindung der allgemeinen Formel I erhält.

Gemäss Verfahrensvariante d) können die Verbindungen der Formel I durch Behandeln mit einer pharmazeutisch annehmbaren anorganischen Säure (z.B. Salzsäure, Bromwasserstoffsäure, Schwefelsäure, Phosphorsäure, etc.) oder mit einer pharmazeutisch annehmbaren organischen Säure (z.B. Essigsäure, Weinsäure, Zitronensäure, Fumarsäure, Maleinsäure, Apfelsäure, Methansulfonsäure, p-Toluolsulfonsäure, etc.) in pharmazeutisch annehmbare Säureadditionssalze übergeführt werden.

Die in Verfahrensvariante a) als Ausgangsstoffe verwendeten Epoxide der allgemeinen Formel II sind neu und ebenfalls Gegenstand der vorliegenden Erfindung. Sie können hergestellt werden, indem man ein Alkalimetall-Derivat eines Phenols der allgemeinen Formel:

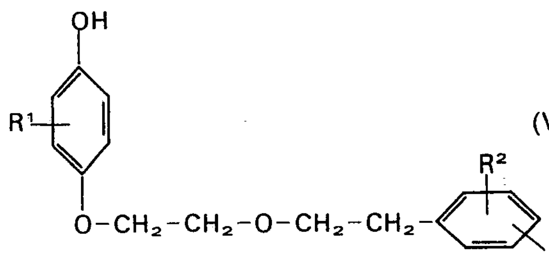

(VI)

worin $R^1$, $R^2$ und $R^3$ obige Bedeutung besitzen, mit Epichlorhydrin oder Epibromhydrin umsetzt.

Die Reaktion eines Alkalimetall-Derivates eines Phenols der Formel VI mit Epichlorhydrin oder Epibromhydrin, vorzugsweise mit Epichlorhydrin, kann in Analogie zur Reaktion eines Alkalimetall-Derivates eines Phenols der Formel IV mit einer Verbindung der Formel V durchgeführt werden.

Die Phenole der obigen allgemeinen Formel VI sind neu und ebenfalls Gegenstand der vorliegenden Erfindung. Sie können hergestellt werden, indem man ein Alkalimetall-Derivat eines Phenols der allgemeinen Formel:

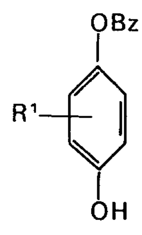

(VII)

worin $R^1$ obige Bedeutung besitzt, und Bz Benzyl bedeutet, mit einer Verbindung der obigen allgemeinen Formel V umsetzt und die erhaltene Verbindung der allgemeinen Formel:

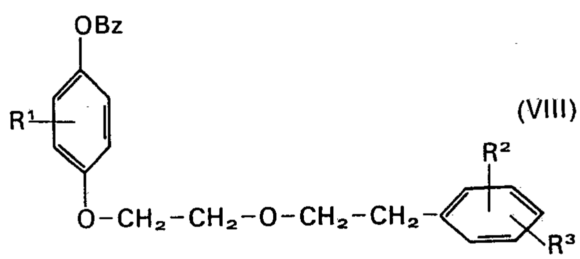

(VIII)

worin $R^1$, $R^2$, $R^3$ und Bz obige Bedeutung besitzen, debenzyliert.

Die Reaktion eines Alkalimetall-Derivates eines Phenols der allgemeinen Formel VII mit einer Verbindung der allgemeinen Formel V kann in Analogie zur Reaktion eines Alkalimetall-Derivates eines Phenols der allgemeinen Formel IV mit einer Verbindung der allgemeinen Formel V durchgeführt werden.

Die Debenzylierung einer Verbindung der allgemeinen Formel VIII kann in an sich bekannter Weise durchgeführt werden; beispielsweise unter Verwendung von Wasserstoff in Gegenwart eines Katalysators (z.B. Palladium/Kohle oder dergleichen) oder unter Verwendung von Bromwasserstoff in Eisessig.

Die Phenole der allgemeinen Formel VII, sofern sie noch nicht bekannt sind, können wie in den nachfolgenden Beispielen beschrieben oder in Analogie dazu hergestellt werden.

Die Verbindungen der allgemeinen Formel VIII sind neu und ebenfalls Gegenstand der vorliegenden Erfindung.

Phenole der allgemeinen Formel VI, worin $R^2$ und $R^3$ je Wasserstoff, Halogen, niederes Alkyl oder niederes Alkoxy bedeuten, können auch dadurch hergestellt werden, dass man ein Alkalimetall-Derivat eines Phenols der allgemeinen Formel:

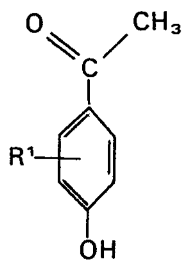

(IX)

worin $R^1$ obige Bedeutung besitzt, mit einer Verbindung der obigen allgemeinen Formel V, worin $R^2$ und $R^3$ je Wasserstoff, Halogen, niederes Alkyl oder niederes Alkoxy bedeuten, umsetzt und in der erhaltenen Verbindung der allgemeinen Formel:

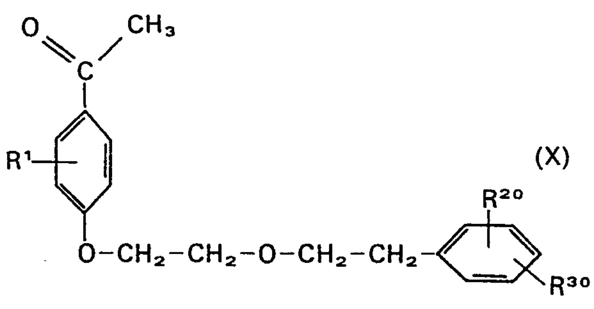

(X)

worin $R^1$ obige Bedeutung besitzt und $R^{20}$ und $R^{30}$ je Wasserstoff, Halogen, niederes Alkyl oder niederes Alkoxy bedeuten, die Acetylgruppe durch Hydroxy ersetzt.

Die Reaktion eines Alkalimetall-Derivates eines Phenols der allgemeinen Formel IX mit einer Verbindung der allgemeinen Formel V kann in Analogie zur Reaktion eines Alkalimetall-Derivates eines Phenols der allgemeinen Formel IV mit einer Verbindung der allgemeinen Formel V durchgeführt werden.

Der Ersatz der Acetylgruppe in einer Verbindung der allgemeinen Formel X durch Hydroxy kann in an sich bekannter Weise erfolgen; beispielsweise durch Oxydation mit einer organischen Persäure, wie m-Chlorperbenzoesäure, zweckmässigerweise in einem inerten organischen Lösungsmittel, beispielsweise in einem halogenierten Kohlenwasserstoff (z.B. Methylenchlorid), und nachfolgender Behandlung mit einem Alkalimetallniederalkoxyd in einem entsprechenden niederen Alkohol (z.B. Natriumäthoxyd in Äthanol).

Die Phenole der allgemeinen Formel IX, sofern sie noch nicht bekannt sind, können wie in den nachfolgenden Beispielen beschrieben oder in Analogie dazu hergestellt werden.

Die Verbindungen der allgemeinen Formel X sind neu und ebenfalls Gegenstand der vorliegenden Erfindung.

Die als Ausgangsstoffe verwendeten Phenole der allgemeinen Formel IV sind bekannte Verbindungen, oder können in Analogie zu den bekannten Verbindungen in an sich bekannter Weise hergestellt werden.

Die als Ausgangsstoffe verwendeten Verbindungen der allgemeinen Formel V gehören einer an sich bekannten Stoffklasse an und können beispielsweise hergestellt werden, indem man zuerst einen Alkohol der allgemeinen Formel:

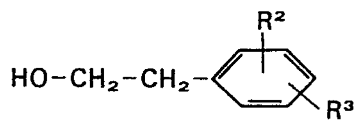

(XI)

worin $R^2$ und $R^3$ obige Bedeutung besitzen, mit Chloressigsäure umsetzt und somit eine Säure der allgemeinen Formel:

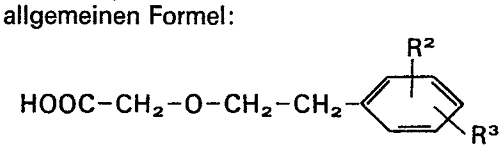

(XII)

worin $R^2$ und $R^3$ obige Bedeutung besitzen, erhält.

Die Reaktion eines Alkohols der allgemeinen Formel XI (das eine bekannte Verbindung ist oder in Analogie zur Herstellung der bekannten Verbindungen in an sich bekannter Weise hergestellt werden kann) mit Chloressigsäure kann in einem inerten organischen Lösungsmittel, wie Dimethylformamid oder Dimethylsulfoxyd, und in Gegenwart einer Alkalimetall-Base, wie einem Alkalimetallhydrid (z.B. Natriumhydrid) und bei erhöhter Temperatur (z.B. etwa 60-120°C) durchgeführt werden.

Eine so erhaltene Säure der allgemeinen Formel XII wird anschliessend zu einem Alkohol der allgemeinen Formel:

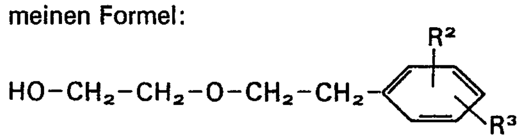

(XIII)

worin $R^2$ und $R^3$ obige Bedeutung besitzen, reduziert.

Die Reduktion einer Säure der allgemeinen Formel XII wird in an sich bekannter Weise durchgeführt; beispielsweise unter Verwendung von Lithiumaluminiumhydrid in einem inerten organischen Lösungsmittel, wie Tetrahydrofuran, und bei etwa Raumtemperatur.

Schliesslich wird ein Alkohol der allgemeinen Formel XIII durch Sulphonylierung in eine Verbindung der allgemeinen Formel V übergeführt. Diese Sulphonylierung wird in an sich bekannter Weise durchgeführt; beispielsweise durch Reaktion mit einem niederen Alkylsulfonyl- oder Arylsulfonyhalogenid (z.B. Methansulfonylchlorid, etc.) in Gegenwart einer tertiären organischen Base, wie Triäthylamin oder Pyridin, bei etwa Raumtemperatur.

Die erfindungsgemässen substituierten Phenoxy-amino-propanol-Derivate besitzen kardioselektive β-adrenergische Blockereigenschaften und können demnach bei der Bekämpfung bzw. Verhütung von Herzkrankheiten, wie beispielsweise Angina pectoris und Herzrhythmusstörungen, verwendet werden. Sie können auch als antihypertensive Wirkstoffe verwendet werden.

Die β-adrenergischen Blockereigenschaften der vorliegenden substituierten Phenoxy-aminopropanol-Derivate an $\beta_1$- und $\beta_2$-Adrenalin-Rezeptoren können mittels Standardtestverfahren nachgewiesen werden. In einem solchen Testverfahren werden diese Eigenschaften an Ratten dadurch gezeigt, dass man diejenige Dosierung der zu testenden Verbindung in µg/kg i.v. ermittelt, welche notwendig ist, um die durch Isoprenalin induzierte Tachykardie [$ED_{50}$(HR)] und die durch Isoprenalin induzierte Blutdrucksenkungsreaktion [$ED_{50}$(BD)] um 50% zu reduzieren. Ist für eine getestete Verbindung die $ED_{50}$(BD) signifikant höher als die $ED_{50}$(HR) so blockiert diese Verbindung selektiver die $\beta_1$-Adrenalin-Rezeptoren als die $\beta_2$-Adrenalin-Rezeptoren (d.h. sie ist kardioselektiv).

Die im vorliegenden Test mit repräsentativen Vertretern der erfindungsgemässen, substituierten Phenoxyaminopropanol-Derivate und dem Atenolol (ein allgemein bekanntes und häufig verwendetes kardioselektives β-adrenergisches Blockermittel) erhaltenen Resultate sind in der folgenden Tabelle zusammengefasst:

*Tabelle*

| Derivat | $ED_{50}$ (HT) (µg/kg i.v.) | $ED_{50}$ (BD) (µg/kg i.v.) |
|---|---|---|
| 1-Isopropylamino-3-[4-(2-phenäthyloxy-äthoxy)phenoxy]-2-propanol-hydrochlorid | 15 | >2000 |
| 1-t-Butylamino-3-[4-(2-phenäthyloxy-äthoxy)phenoxy]-2-propanol-hydrochlorid | 18 | 979 |
| 1-/2-Chlor-4-[2-(phenäthyloxy)-äthoxy]phenoxy/-3-isopropylamino-2-propanol-hydrochlorid | 25 | 834 |
| 1-/4-[2-(4-Fluor-phenäthyloxy)-äthoxy]phenoxy/-3-isopropylamino-2-propanol-hydrochlorid | 3 | >2000 |
| Atenolol | 91 | 2130 |

Die erfindungsgemässen substituierten Phenoxy-amino-propanol-Derivate können als Arzneimittel in Form von pharmazeutischen Präparaten, welche diese in Kombination mit einem verträglichen pharmazeutischen Trägermaterial enthalten können, verwendet werden. Dieses Trägermaterial kann ein inertes organisches oder anorganisches Trägermaterial sein, das für die enterale (z.B. orale) oder parenterale Verabreichung geeignet ist. Als Beispiele solcher Trägermaterialien seien Wasser, Gelatine, Lactose, Stärke, Magnesiumstearat, Talk, Gummi arabicum, Polyalkylenglykole, pflanzliche Öle, Vaselin und dergleichen genannt. Die pharmazeutischen Präparate können in herkömmlicher Weise hergestellt werden und in festen Dosierungsformen (z.B. als Tabletten, Dragées, Suppositorien oder Kapseln) oder in flüssigen Dosierungsformen (z.B. als Lösungen, Suspensionen oder Emulsionen) vorliegen. Die pharmazeutischen Präparate können herkömmlichen pharmazeutischen Nachbehandlungsmassnahmen, wie Sterilisation, unterworfen werden. Weiterhin können die pharmazeutischen Präparate herkömmliche Hilfsmittel, wie Konservierungsmittel, Stabilisierungsmittel, Emulgiermittel, Aromastoffe, Netzmittel, Salze, um den osmotischen Druck zu verändern, Puffer und dergleichen enthalten.

Die erfindungsgemässen, substituierten Phenoxy-amino-propanol-Derivate können Erwachsenen in einer täglichen Dosierung von etwa 1 mg/kg bis 10 mg/kg in einer einzigen Dosis oder in mehreren Dosen verabreicht werden. Es sei festgehalten, dass die obigen Dosierungsangaben lediglich als Beispiele dienen, und dass die Dosierungen nach oben oder nach unten, in Abhängigkeit von Faktoren, wie das zu verabreichende, substituierte Phenoxy-aminopropanol-Derivat, die Art der Verabreichung und die Bedürfnisse des Patienten, wie sie vom behandelnden Arzt festgestellt werden, variieren können.

Die folgenden Beispiele erläutern das erfindungsgemässe Verfahren.

*Beispiel 1:*

4,5 g (20 mMol) 1-Isopropylamino-3-(4-hydr-

oxyphenoxy)-2-propanol in 20 ml Dimethylformamid werden mit 0,96 g (20 mMol) Natriumhydrid (50-proz. Öldispersion) behandelt. Die erhaltene Mischung wird während 5 Minuten gerührt, mit 4,88 g (20 mMol) 2-(2-Phenyläthoxy)äthylmethansulfonat versetzt und anschliessend während 0,5 Stunden unter Rühren auf 60°C erhitzt. Man dampft die Mischung zur Trockene ein und verteilt den Rückstand zwischen 2N Natronlauge und Methylenchlorid. Die organische Phase wird abgetrennt, gut mit Wasser gewaschen, über Natriumsulfat getrocknet, filtriert und eingedampft. Man nimmt den Rückstand in 20 ml Äthanol auf, sättigt die erhaltene Lösung mit Chlorwasserstoff und dampft zur Trockene ein. Der kristalline Rückstand liefert nach Umkristallisieren aus Isopropanol 4,2 g (51%) 1-Isopropylamino-3-[4-(2-phenäthyloxyäthoxy)phenoxy]-2-propanol-hydrochlorid vom Schmelzpunkt 105-107°C.

Das als Ausgangsmaterial verwendete 2-(2-Phenyläthoxy)-äthyl-methansulfonat kann wie folgt hergestellt werden:

a) 6,1 g (50 mMol) Phenäthylalkohol in 100 ml Dimethylformamid werden mit 4,8 g (100 mMol) Natriumhydrid (50%ige Öldispersion) behandelt. Man rührt die Mischung während 10 Minuten bei 60°C, versetzt mit 4,73 g (50 mMol) Chloressigsäure und erhitzt unter Rühren während 0,5 Stunden auf 60°C. Man dampft zur Trockene ein und verteilt den Rückstand zwischen Wasser und Diäthyläther. Die wässerige Phase wird mit konzentrierter Salzsäure auf pH 1 gestellt und mit Essigester extrahiert. Die organische Phase wird gut mit Wasser gewaschen, über Natriumsulfat getrocknet, filtriert und zur Trockene eingedampft. Der Rückstand liefert nach Umkristallisieren aus Essigester/Hexan 7,65 g (85%) 2-Phenyläthoxyessigsäure vom Schmelzpunkt 46-48°C.

b) Man löst das unter a) erhaltene Material in 50 ml Tetrahydrofuran, gibt die erhaltene Lösung über einen Zeitraum von 10 Minuten tropfenweise zu einer gerührten und in einem Eisbad gekühlten Suspension von 1,62 g (42,5 mMol) Lithiumaluminiumhydrid in 50 ml Tetrahydrofuran und rührt die erhaltene Mischung während 0,5 Stunden bei Raumtemperatur. Man zerstört das überschüssige Lithiumaluminiumhydrid durch Zugabe von 1,6 ml Wasser, 1,6 ml 15-proz. wässeriger Natronlauge und schliesslich 5 ml Wasser. Die anorganischen Festkörper werden abfiltriert und gut mit Diäthyläther gewaschen. Durch Eindampfen der Filtrate erhält man 6,50 g (92%) 2-(2-Phenyläthoxy)-äthanol als Öl, das chromatographisch einheitlich ist. Dieses Produkt kann durch Destillation unter vermindertem Druck gereinigt werden; Siedepunkt 140-142°C/15 mmHg.

c) Man löst das unter b) erhaltene Material in 100 ml Pyridin, behandelt die Lösung mit 4,48 g (39 mMol) Methansulfonylchlorid und rührt die erhaltene Mischung während 0,5 Stunden bei Raumtemperatur. Man dampft ein und verteilt den Rückstand zwischen Essigester und 2N Salzsäure. Die organische Phase wird mit Wasser gewaschen, über Natriumsulfat getrocknet, filtriert und eingedampft. Man erhält 8,1 g (85%) 2-(2-Phenyläthoxy)äthyl-methansulfonat als Öl, das chromatographisch einheitlich ist.

*Beispiel 2:*

In Analogie zu den Angaben im ersten Absatz von Beispiel 1 erhält man aus 1,69 g 1-Isopropylamino-3-(4-hydroxyphenoxy)-2-propanol und 1,97 g 2-[2-(4-Fluorphenyl)-äthoxy]äthylmethansulfonat 1,6 g (50%) 1-/4-[2-(4-Fluorphenäthyloxy)äthoxy]phenoxy/-3-isopropylamino-2-propanol-hydrochlorid vom Schmelzpunkt 88-92°C (aus Isopropanol).

Das als Ausgangsmaterial verwendete 2-[2-(4-Fluorphenyl)äthoxy]äthyl-methansulfonat kann wie folgt hergestellt werden:

a) 7,0 g (50 mMol) 4-Fluorphenäthylalkohol werden in Analogie zu den Angaben in Beispiel 1 a) mit 4,73 g (50 mMol) Chloressigsäure umgesetzt. Man erhält 7,8 g (79%) 2-(4-Fluorphenyl)äthoxyessigsäure vom Schmelzpunkt 82-85°C (aus Methylcyclohexan).

b) Das unter a) erhaltene Material wird in Analogie zu den Angaben in Beispiel 1 b) mit Lithiumaluminiumhydrid reduziert. Man erhält 6,6 g (91%) 2-[2-(4-Fluorphenyl)äthoxy]äthanol als Öl, das chromatographisch einheitlich ist.

c) Das unter b) erhaltene Material wird in Analogie zu den Angaben in Beispiel 1 c) mit Methansulfonylchlorid sulfonyliert. Man erhält 8,2 g (87%) 2-[2-(4-Fluorphenyl)äthoxy]äthyl-methansulfonat als Öl, das chromatographisch einheitlich ist.

*Beispiel 3:*

In Analogie zu den Angaben im ersten Absatz von Beispiel 1 erhält man aus 1,69 g 1-Isopropylamino-3-(4-hydroxyphenoxy)-2-propanol und 1,94 g 2-[2-(4-Methylphenyl)-äthoxy]äthylmethansulfonat 1,6 g (50%) 1-/4-[2-(4-Methylphenäthyloxy)äthoxy]phenoxy/-3-isopropylamino-2-propanolhydrochlorid vom Schmelzpunkt 104-106°C (aus Isopropanol).

Das als Ausgangsmaterial verwendete 2-[2-(4-Methylphenyl)äthoxy]äthyl-methansulfonat kann wie folgt hergestellt werden:

a) 6,8 g (50 mMol) 4-Methylphenäthylalkohol werden in Analogie zu den Angaben in Beispiel 1 a) mit 4,73 g (50 mMol) Chloressigsäure umgesetzt. Man erhält 8,1 g (84%) 2-(4-Methylphenyl)äthoxyessigsäure vom Schmelzpunkt 65-67°C (aus Methylcyclohexan).

b) Das unter a) erhaltene Material wird in Analogie zu den Angaben in Beispiel 1 b) mit Lithiumaluminiumhydrid reduziert. Man erhält 6,6 g (88%) 2-[2-(4-Methylphenyl)-äthoxy]äthanol als Öl, das chromatographisch einheitlich ist.

c) Das unter b) erhaltene Material wird in Analogie zu den Angaben in Beispiel 1 c) mit Methansulfonylchlorid sulfonyliert. Man erhält 7,8 g (83%) 2-[2-(4-Methylphenyl)-äthoxy]äthylmethansulfonat als Öl, das chromatographisch einheitlich ist.

*Beispiel 4:*

In Analogie zu den Angaben im ersten Absatz

von Beispiel 1 erhält man aus 1,69 g 1-Isopropylamino-3-(4-hydroxyphenoxy)-2-propanol und 2,16 g 2-[2-(4-Methylthiophenyl)äthoxy]äthyl-methansulfonat 1,40 g (41%) 1-/4-[2-(4-Methylthiophenäthyloxy)äthoxy]phenoxy/-3-isopropylamino-2-propanol-hydrochlorid vom Schmelzpunkt 98-101°C (aus Essigester).

Das als Ausgangsmaterial verwendete 2-[2-(4-Methylthiophenyl)äthoxy]äthyl-methansulfonat kann wie folgt hergestellt werden:

a) Man behandelt 6,27 g (37 mMol) 4-Methylthiophenäthylalkohol in 100 ml Dimethylsulfoxyd mit 3,58 g (74 mMol) Natriumhydrid (50-proz. Öldispersion), rührt die erhaltene Mischung während 10 Minuten bei 60°C, versetzt mit 3,53 g (37 mMol) Chloressigsäure und erhitzt anschliessend noch während 3 Stunden unter Rühren auf 80°C. Man kühlt ab, giesst auf Wasser und wäscht mit Essigester. Die wässerige Phase wird mit konzentrierter Salzsäure auf pH 1 gestellt und mit Essigester ausgeschüttelt. Der organische Auszug wird abgetrennt, mit Wasser gut gewaschen, über Natriumsulfat getrocknet, filtriert und zur Trockene eingedampft. Der Rückstand wird aus Toluol/Hexan umkristallisiert und liefert 3,89 g (46%) 2-(4-Methylthiophenyl)äthoxyessigsäure vom Schmelzpunkt 58-60°C.

b) Das unter a) erhaltene Material wird in Analogie zu den Angaben in Beispiel 1 b) mit Lithiumaluminiumhydrid reduziert. Man erhält 1,97 g (54%) 2-[2-(4-Methylthiophenyl)äthoxy]äthanol als Öl, das chromatographisch einheitlich ist.

c) Das unter b) erhaltene Material wird in Analogie zu den Angaben in Beispiel 1 c) mit Methansulfonylchlorid sulfonyliert. Man erhält 2,41 g (90%) 2-[2-(4-Methylthiophenyl)äthoxy]äthyl-methansulfonat als Öl, das chromatographisch einheitlich ist.

*Beispiel 5:*

In Analogie zu den Angaben im ersten Absatz von Beispiel 1, jedoch unter Verwendung von 1-t-Butylamino-3-(4-hydroxyphenoxy)-2-propanol anstelle von 1-Isopropylamino-3-(4-hydroxyphenoxy)-2-propanol, erhält man 1-t-Butylamino-3-[4-(2-phenäthyloxyäthoxy)phenoxy]-2-propanol-hydrochlorid vom Schmelzpunkt 87-88°C (aus Äthanol/Diäthyläther).

*Beispiel 6:*

Man löst 3,09 g (12 mMol) 4-(2-Phenäthyloxyäthoxy)-phenol in 50 ml Dimethylformamid und rührt die Lösung zusammen mit 0,58 g (12 mMol) Natriumhydrid (50-proz. Öldispersion) während 5 Minuten. Man versetzt anschliessend mit 10 ml Epichlorhydrin, rührt während 0,5 Stunden bei 60°C, entfernt das Lösungsmittel und überschüssiges Epichlorhydrin unter vermindertem Druck und verteilt den Rückstand zwischen Essigester und Wasser. Die organische Phase wird abgetrennt, über Natriumsulfat getrocknet, filtriert und eingedampft. Man erhält 3,2 g Epoxid, das ohne weitere Reinigung in 50 ml Äthanol, das 15 ml Isoproplyamin enthält, aufgenommen wird. Die Mischung wird anschliessend über Nacht bei Raumtemperatur stehengelassen. Man dampft zur Trockene ein. Aus dem Rückstand erhält man 2,7 g (59%) 1-Isopropylamino-3-[4-(2-phenäthyloxyäthoxy)-phenoxy]-2-propanol-hydrochlorid. Dieses Material ist mit dem im ersten Absatz von Beispiel 1 erhaltenen identisch.

Das als Ausgangsmaterial verwendete 4-(2-Phenäthyloxyäthoxy)phenol kann wie folgt hergestellt werden:

a) Man behandelt 10 g (50 mMol) 4-Benzyloxyphenol in 120 ml Dimethylformamid mit 2,4 g (50 mMol) Natriumhydrid (50-proz. Öldispersion), rührt die erhaltene Mischung während 5 Minuten, versetzt mit 12,2 g (50 mMol) 2-(2-Phenyläthoxy)äthyl-methansulfonat [hergestellt wie in Beispiel 1 a) beschrieben] und rührt die Mischung während 0,5 Stunden bei 60°C. Man dampft zur Trockene ein und verteilt den Rückstand zwischen 2N Natronlauge und Essigester. Die organische Phase wird abgetrennt, gut mit Wasser gewaschen, über Natriumsulfat getrocknet, filtriert und eingedampft. Der Rückstand wird aus Methylcyclohexan umkristallisiert und liefert 16,1 g (93%) 1-Benzyloxy-4-(2-phenäthyloxyäthoxy)benzol vom Schmelzpunkt 46-49°C.

b) Man löst das unter a) erhaltene Material in 200 ml Äthanol und 200 ml Essigester und hydriert über Nacht bei Atmosphärendruck und Raumtemperatur in Gegenwart von 0,4 g 10-proz. Palladium/Kohle. Der Katalysator wird abfiltriert und das Filtrat wird eingedampft. Man erhält 10,8 g (91%) 4-(2-Phenäthyloxyäthoxy)phenol als Öl, das chromatographisch einheitlich ist.

*Beispiel 7:*

In Analogie zu den Angaben im ersten Absatz von Beispiel 6 erhält man aus 2-Chlor-4-(2-phenäthyloxyäthoxy)-phenol 1-/2-Chlor-4-[2-(phenäthyloxy)äthoxy]phenoxy/-3-isopropylamino-2-propanol-hydrochlorid vom Schmelzpunkt 82-84°C (aus Isopropanol).

Das als Ausgangsmaterial verwendete 2-Chlor-4-(2-phenäthyloxyäthoxy)phenol kann wie folgt hergestellt werden:

a) Man löst 8,9 g (36 mMol) 3-Chlor-4-benzyloxybenzaldehyd in 150 ml Methylenchlorid, das 7,3 g (36 mMol) m-Chlorperbenzoesäure enthält und rührt die erhaltene Lösung über Nacht bei Raumtemperatur. Die Mischung wird zuerst mit gesättigter Natriumbicarbonatlösung und Wasser gewaschen. Die organische Phase wird abgetrennt, über Natriumsulfat getrocknet, filtriert und zur Trockene eingedampft. Man löst den Rückstand in 100 ml Äthanol, das 2,5 g (36 mMol) Natriumäthoxyd enthält, und rührt die Lösung während 1 Stunde bei Raumtemperatur. Nach Entfernen des Lösungsmittels durch Eindampfen wird der Rückstand mit 2N Salzsäure sauer gestellt und anschliessend mit Essigester extrahiert. Die organische Phase wird abgetrennt, mit Wasser gewaschen, über Natriumsulfat getrocknet, filtriert und zur Trockene eingedampft. Umkristallisieren des kristallinen Rückstandes aus Hexan liefert 5,1 g (60%) 3-Chlor-4-benzyloxyphenol vom Schmelzpunkt 49-51°C.

b) In Analogie zu den Angaben in Beispiel 6 a) erhält man aus 4,12 g 3-Chlor-4-benzyloxyphenol ein Rohprodukt, das nach Reinigung durch Säulenchromatographie an Kieselgel unter Eluieren mit Chloroform 4,30 g (64%) 1-Benzyloxy-2-chlor-4-(2-phenäthyloxyäthoxy)benzol als Öl liefert, das chromatographisch einheitlich ist.

c) Das unter b) erhaltene 1-Benzyloxy-2-chlor-4-(2-phenäthyloxyäthoxy)benzol wird während 30 Minuten bei 25°C in 20 ml 48-proz. Bromwasserstoff in Eisessig gerührt. Man dampft die Lösung zur Trockene ein und verteilt den Rückstand zwischen 2N Natronlauge und Diäthyläther. Die wässerige Phase wird mit konzentrierter Salzsäure auf pH 6 gestellt und mit Essigester extrahiert. Die organische Phase wird mit Wasser gewaschen, über Natriumsulfat getrocknet, filtriert und eingedampft. Man erhält 2,06 g (76%) 2-Chlor-4-(2-phenäthyloxyäthoxy)phenol als Öl, das chromatographisch einheitlich ist.

*Beispiel 8:*

In Analogie zu den Angaben im ersten Absatz von Beispiel 6 erhält man aus 2-Methyl-4-(2-phenäthyloxyäthoxy)-phénol 1-/2-Methyl-4-[2-(phenäthyloxy)äthoxy]phenoxy/-3-isopropylamino-2-propanol-hydrochlorid vom Schmelzpunkt 85-87°C (aus Essigester).

Das als Ausgangsmaterial verwendete 2-Methyl-4-(2-phenäthyloxyäthoxy)phenol kann wie folgt hergestellt werden:

a) In Analogie zu den Angaben in Beispiel 6 a) erhält man aus 3,0 g 4-Hydroxy-2-methylacetophenon 5,90 g (99%) 2-Methyl-4-(2-phenäthyloxyäthoxy)acetophenon als Öl, das chromatographisch einheitlich ist.

b) Man löst das unter a) erhaltene 2-Methyl-4-(2-phenäthyloxyäthoxy)acetophenon in 80 ml Methylenchlorid, das 3,91 g (20 mMol) m-Chlorperbenzoesäure enthält, lässt die Lösung während 5 Tagen bei Raumtemperatur stehen, wäscht die Lösung zuerst mit gesättigter Natriummetabisulfitlösung und anschliessend mit gesättigter Natriumbicarbonatlösung, trocknet über Natriumsulfat, filtriert und dampft ein. Man nimmt das Rohprodukt in 250 ml Methanol, das 2,25 g Natriummethoxyd enthält, auf und lässtes während 1 Stunde bei Raumtemperatur stehen. Man entfernt das Lösungsmittel unter vermindertem Druck und verteilt den Rückstand zwischen verdünnter Salzsäure und Methylenchlorid. Die organische Phase wird abgetrennt, mit Wasser gewaschen, über Natriumsulfat getrocknet, filtriert und eingedampft. Der Rückstand wird durch Chromatographieren an Kieselgel unter Eluieren mit 40% Chloroform/Hexan gereinigt. Durch Eindampfen des Eluates erhält man 3,3 g (61%) 2-Methyl-4-(2-phenäthyloxyäthoxy)phenol als Öl, das chromatographisch einheitlich ist.

*Beispiel 9:*

In Analogie zu den Angaben im ersten Absatz von Beispiel 1 erhält man aus 2,25 g 1-Isopropylamino-3-(4-hydroxyphenoxy)-2-propanol und 2,74 g 2-[2-(4-Methoxyphenyl)äthoxy]äthylmethansulfonat 3,4 g (77%) 1-/4-[2-(4-Methoxyphenäthyloxy)äthoxy]phenoxy/-3-isopropylamino-2-propanol-hydrochlorid vom Schmelzpunkt 99-101°C (aus Isopropanol).

Das als Ausgangsmaterial verwendete 2-[2-(4-Methoxyphenyl)äthoxy]äthyl-methansulfonat kann wie folgt hergestellt werden:

a) 6,08 g 4-Methoxyphenäthylalkohol werden in Analogie zu den Angaben in Beispiel 4 a) mit 3,8 g Chloressigsäure umgesetzt. Man erhält 6,64 g (79%) 2-(4-Methoxyphenyl)-äthoxyessigsäure vom Schmelzpunkt 82-84°C (aus Toluol).

b) Die unter a) erhaltene 2-(4-Methoxyphenyl)äthoxyessigsäure wird in Analogie zu den Angaben in Beispiel 1 b) mit Lithiumaluminiumhydrid reduziert. Man erhält 5,4 g (96%) 2-[2-(4-Methoxyphenyl)äthoxy]äthanol als Öl, das chromatographisch einheitlich ist.

c) Das unter b) erhaltene 2-[2-(4-Methoxyphenyl)äthoxy]-äthanol wird in Analogie zu den Angaben in Beispiel 1 c) mit Methansulfonylchlorid sulfonyliert. Man erhält 8,05 g (100%) 2-[2-(4-Methoxyphenyl)äthoxy]äthyl-methansulfonat als Öl, das chromatographisch einheitlich ist.

*Beispiel 10:*

In Analogie zu den Angaben im ersten Absatz von Beispiel 1 erhält man aus 1,08 g 1-Isopropylamino-3-(4-hydroxyphenoxy)-2-propanol und 1,42 g 2-[2-(4-Chlorphenyl)äthoxy]äthyl-methansulfonat 1,3 g (57%) 1-/4-[2-(4-Chlorphenäthyloxy)äthoxy]phenoxy/-3-isopropylamino-2-propanol-hydrochlorid vom Schmelzpunkt 99-101°C (aus Essigester).

Das als Ausgangsmaterial verwendete 2-[2-(4-Chlorphenyl)äthoxy]äthyl-methansulfonat kann wie folgt hergestellt werden:

a) 3,15 g 4-Chlorphenäthylalkohol werden in Analogie zu den Angaben in Beispiel 1 a) mit 2,2 g Chloressigsäure umgesetzt. Man erhält 4,55 g (91%) 2-(4-Chlorphenyl)äthoxy-essigsäure vom Schmelzpunkt 75-77°C (aus Essigester/Hexan).

b) Die unter a) erhaltene 2-(4-Chlorphenyl)-äthoxyessigsäure wird in Analogie zu den Angaben in Beispiel 1 b) mit Lithiumaluminiumhydrid reduziert. Man erhält 1,6 g (43%) 2-[2-(4-Chlorphenyl)äthoxy]äthanol als Öl, das chromatographisch einheitlich ist.

c) Das unter b) erhaltene 2-[2-(4-Chlorphenyl)äthoxy]äthanol wird in Analogie zu den Angaben in Beispiel 1 c) mit Methansulfonylchlorid sulfonyliert. Man erhält 1,5 g (92%) 2-[2-(4-Chlorphenyl)äthoxy]äthyl-methansulfonat als Öl, das chromatographisch einheitlich ist.

*Beispiel 11:*

In Analogie zu den Angaben im ersten Absatz von Beispiel 6 erhält man aus 2-Fluor-4-[2-(4-fluorphenäthyloxy)äthoxy]phenol 1-/2-Fluor-4-[2-(4-fluorphenäthyloxy)äthoxy]phenoxy/-3-isopropylamino-2-propanol-p-toluolsulfonat vom Schmelzpunkt 77-80°C (aus Isopropanol).

Das als Ausgangsmaterial verwendete 2-Fluor-4-[2-(4-fluorphenäthyloxy)äthoxy]phenol kann wie folgt hergestellt werden:

a) Man behandelt 4,32 g (28 mMol) 3-Fluor-4-hydroxyacetophenon in 50 ml Dimethylformamid mit 1,35 g (28 mMol) Natriumhydrid (50-proz. Öldispersion), rührt die Mischung während 5 Minuten, versetzt mit 4,79 g (28 mMol) Benzylbromid und erhitzt die Mischung während 0,5 Stunden unter Rühren auf 60°C. Man dampft zur Trockene ein und verteilt den Rückstand zwischen 1 N Natronlauge und Essigester. Die organische Phase wird abgetrennt, mit Wasser gut gewaschen, über Natriumsulfat getrocknet, filtriert und eingedampft. Umkristallisieren des gelben Festkörpers aus Essigester/Petroläther liefert 4,99 g (73%) 3-Fluor-4-benzyloxyacetophenon vom Schmelzpunkt 82-85°C.

b) Das unter a) erhaltene substituierte Acetophenon wird in 100 ml Methylenchlorid, das 4,12 g (20,3 mMol) m-Chlorperbenzoesäure enthält, aufgelöst. Man lässt die Lösung während 5 Tagen bei Raumtemperatur stehen, wäscht mit gesättigter Natriummetabisulfitlösung und anschliessend mit gesättigter Natriumbicarbonatlösung, trocknet über Natriumsulfat, filtriert und dampft ein. Man nimmt das Rohprodukt in 100 ml Methanol, das 1,2 g Natriummethoxyd enthält, auf und lässt die Mischung bei Raumtemperatur während 5 Minuten stehen. Man entfernt das Lösungsmittel unter vermindertem Druck und verteilt den Rückstand zwischen verdünnter Salzsäure und Essigester. Die organische Phase wird abgetrennt, mit Wasser gewaschen, über Natriumsulfat getrocknet, filtriert und eingedampft. Kristallisieren des Rückstandes aus Methylcyclohexan liefert 2,53 g (57%) 3-Fluor-4-benzyloxyphenol vom Schmelzpunkt 80-82°C.

c) In Analogie zu den Angaben in Beispiel 6 a) erhält man aus 2,42 g 3-Fluor-4-benzyloxyphenol und 2,91 g 2-[2-(4-Fluorphenyl)äthoxy]äthyl-methansulfonat [hergestellt wie in Beispiel 2 a) beschrieben] 4,23 g (99%) 1-Benzyloxy-2-fluor-4-[2-(4-fluorphenäthyloxy)äthoxy]benzol als Öl, das chromatographisch einheitlich ist.

d) Aus dem unter c) erhaltenen 1-Benzyloxy-2-fluor-4-[2-(4-fluorphenäthyloxy)äthoxy]benzol erhält man in Analogie zu den Angaben in Beispiel 6 b) 2,95 g (91%) 2-Fluor-4-[2-(4-fluorphenäthyloxy)äthoxy]phenol als Öl, das chromatographisch einheitlich ist.

*Beispiel 12:*

In Analogie zu den Angaben im ersten Absatz von Beispiel 6 erhält man aus 3-Fluor-4-[2-(4-fluorphenäthyloxy)äthoxy]phenol 1-/3-Fluor-4-[2-(4-fluorphenäthyloxy)äthoxy]-phenoxy/-3-isopropylamino-2-propanol-hydrochlorid vom Schmelzpunkt 74-76°C (aus Isopropanol).

Das als Ausgangsmaterial verwendete 3-Fluor-4-[2-(4-fluorphenäthyloxy)äthoxy]phenol kann wie folgt hergestellt werden:

a) In Analogie zu den Angaben in Beispiel 6 a) erhält man aus 1,21 g 3-Fluor-4-hydroxyacetophenon und 2,06 g 2-[2-(4-Fluorphenyl)äthoxy]äthyl-methansulfonat [hergestellt wie in Beispiel 2 a) beschrieben] 2,18 g (87%) 3-Fluor-4-[2-(4-fluorphenäthyloxy)äthoxy]acetophenon als Öl, das chromatographisch einheitlich ist.

b) Man löst das unter a) erhaltene substituierte Acetophenon in 30 ml Methylenchlorid, das 1,38 g (6,8 mMol) m-Chlorperbenzoesäure enthält, lässt die Lösung während 5 Tagen bei Raumtemperatur stehen, wäscht mit gesättigter Natriummetabisulfitlösung und anschliessend mit gesättigter Natriumbicarbonatlösung, trocknet über Natriumsulfat, filtriert und dampft ein. Man nimmt das Rohprodukt in 50 ml Methanol, das 0,4 g Natriummethoxyd enthält, auf und lässt die Mischung während 1 Stunde bei Raumtemperatur stehen. Man entfernt das Lösungsmittel unter vermindertem Druck und verteilt den Rückstand zwischen verdünnter Salzsäure und Methylenchlorid. Die organische Phase wird abgetrennt, mit Wasser gewaschen, über Natriumsulfat getrocknet, filtriert und eingedampft. Der Rückstand wird an Kieselgel unter Eluieren mit 70% Chloroform/Hexan chromatographiert. Nach Eindampfen des Eluates erhält man 1,0 g (50%) 3-Fluor-4-[2-(4-fluorphenäthyloxy)äthoxy]-phenol als Öl, das chromatographisch einheitlich ist.

*Beispiel 13:*

In Analogie zu den Angaben im ersten Absatz von Beispiel 1 erhält man aus 2,52 g 1-Isopropylamino-3-(4-hydroxyphenoxy)-2-propanol und 3,12 g 2-[2-2,4-Difluorphenyl)äthoxy]äthyl-methansulfonat 2,3 g (46%) 1-/4-[2-(2,4-Difluorphenäthyloxy)äthoxy]phenoxy/-3-isopropylamino-2-propanol-hydrochlorid vom Schmelzpunkt 69-72°C (aus Isopropanol/Diäthyläther).

Das als Ausgangsmaterial verwendete 2-[2-(2,4-Difluorphenyl)äthoxy]äthyl-methansulfonat kann wie folgt hergestellt werden:

a) Eine Lösung von 25,7 g (124 mMol) 2,4-Difluorbenzylbromid in 100 ml Tetrahydrofuran wird tropfenweise zu einer gerührten Suspension von 3,0 g (124 mMol) Magnesiumspäne in 30 ml Tetrahydrofuran über einen Zeitraum von 0,5 Stunden gegeben. Nach beendeter Zugabe rührt man noch während 10 Minuten und behandelt anschliessend während 1 Stunde mit gasförmigen Kohlendioxyd. Man dampft zur Trockene ein und verteilt den Rückstand zwischen Diäthyläther und verdünnter Salzsäure. Die Diäthylätherphase wird abgetrennt und mit 2N Natronlauge ausgeschüttelt. Der alkalische Extrakt wird mit konzentrierter Salzsäure sauer gestellt und mit Diäthyläther ausgeschüttelt. Man erhält 5,1 g (24%) rohe 2,4-Difluorphenylessigsäure, welche ohne weitere Reinigung in 25 ml Tetrahydrofuran gelöst wird. Diese Lösung gibt man tropfenweise zu einer gerührten Suspension von 2,0 g Lithiumaluminiumhydrid in 100 ml Tetrahydrofuran. Nach beendeter Zugabe rührt man noch während 1 Stunde und zerstört anschliessend das überschüssige Lithiumaluminiumhydrid durch tropfenweise Zugabe von 2 ml Wasser in 20 ml Tetrahydrofuran gefolgt von 2 ml 15-proz. Natronlauge und 6 ml Wasser. Man filtriert die Suspension unter Verwendung einer Filtrierhilfe und unter gutem Nachwaschen mit

Diäthyläther. Die vereinigten Filtrate werden zur Trockene eingedampft. Man nimmt den Rückstand in Methylenchlorid auf, trocknet über Natriumsulfat, filtriert und dampft ein. Man erhält 3,95 g (84%) 2,4-Difluorphenäthylalkohol als Öl, das chromatographisch einheitlich ist.

b) 3,63 g (23 mMol) 2,4-Difluorphenäthylalkohol werden in Analogie zu den Angaben in Beispiel 1 a), jedoch unter Erhitzen der Reaktionsmischung auf 120°C während 1 Stunde, mit 2,17 g (23 mMol) Chloressigsäure umgesetzt. Man erhält 2,7 g (54%) 2-(2,4-Difluorphenyl)äthoxyessigsäure als Öl, das chromatographisch einheitlich ist.

c) Die unter b) erhaltene 2-(2,4-Difluorphenyl)äthoxyessigsäure wird in Analogie zu den Angaben in Beispiel 1 b) mit Lithiumaluminiumhydrid reduziert. Man erhält 2,31 g (93%) 2-[2-(2,4-Difluorphenyl)äthoxy]äthanol als Öl, das chromatographisch einheitlich ist.

d) Das unter c) erhaltene 2-[2-(2,4-Difluorphenyl)äthoxy]äthanol wird in 40 ml Methylenchlorid, das 1,13 g Triäthylamin enthält, aufgenommen. Man behandelt die erhaltene Lösung mit 1,28 g Methansulfonylchlorid, rührt während 15 Minuten, und wäscht gut mit Wasser. Die organische Phase wird abgetrennt, über Natriumsulfat getrocknet, filtriert und eingedampft. Man erhält 3,12 g (100%) 2-[2-(2,4-Difluorphenyl)äthoxy]-äthyl-methansulfonat als Öl, das chromatographisch einheitlich ist.

Die folgenden Beispiele betreffen typische pharmazeutische Präparate, enthaltend die erfindungsgemässen substituierten Phenoxy-aminopropanol-Derivate:

*Beispiel A:*

Tabletten, enthaltend die folgenden Bestandteile, können in herkömmlicher Weise hergestellt werden:

| *Bestandteile* | *Per Tablette* |
|---|---|
| Substituiertes Phenoxy-aminopropanol-Derivat | 25 mg |
| Lactose | 103 mg |
| Stärke | 61 mg |
| Magnesiumstearat | 11 mg |
| Gesamtgewicht | 200 mg |

*Beispiel B:*

Kapseln, enthaltend die folgenden Bestandteile, können in herkömmlicher Weise hergestellt werden:

| *Bestandteile* | *Per Kapsel* |
|---|---|
| Substituiertes Phenoxy-aminopropanol-Derivat | 25 mg |
| Lactose | 106 mg |
| Stärke | 20 mg |
| Talk | 9 mg |
| Gesamtgewicht | 160 mg |

Die erhaltene Kapselabfüllmasse wird zweckmässigerweise in Hartgelatinekapseln Nr. 4 abgefüllt.

**Patentansprüche für die Vertragsstaaten:** BE, CH, DE, FR, GB, IT, LI, LU, NL, SE

1. Substituierte Phenoxy-aminopropanol-Derivate der allgemeinen Formel:

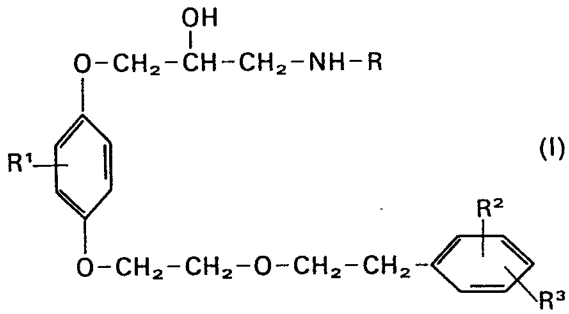

(I)

worin R eine verzweigte, 3 bis 4 Kohlenstoffatome enthaltende Alkylgruppe, $R^1$ Wasserstoff, Halogen oder niederes Alkyl und $R^2$ und $R^3$ je Wasserstoff, Halogen, niederes Alkyl, niederes Alkoxy oder niederes Alkylthio bedeuten, und pharmazeutisch annehmbare Säureadditionssalze davon.

2. Verbindungen gemäss Anspruch 1, dadurch gekennzeichnet, dass $R^1$ in ortho-Stellung zur 3-Alkylamino-2-hydroxypropoxy-gruppe steht und $R^3$ Wasserstoff bedeutet, und pharmazeutisch annehmbare Säureadditionssalze davon.

3. Verbindungen gemäss Anspruch 1 oder 2, dadurch gekennzeichnet, dass R Isopropyl oder t-Butyl bedeutet, und pharmazeutisch annehmbare Säureadditionssalze davon.

4. 1-Isopropylamino-3-[4-(2-phenäthyloxyäthoxy)-phenoxy]-2-propanol oder ein pharmazeutisch annehmbares Säureadditionssalz davon.

5. 1-/4-[2-(4-Fluorphenäthyloxy)äthoxy]-phenoxy/-3-isopropylamino-2-propanol oder ein pharmazeutisch annehmbares Säureadditionssalz davon.

6. 1-/4-[2-(4-Methylphenäthyloxy)äthoxy]-phenoxy/-3-isopropylamino-2-propanol, 1-/4-[2-(4-Methylthiophenäthyloxy)äthoxy]phenoxy/-3-isopropylamino-2-propanol, 1-t-Butylamino-3-[4-(2-phenäthyloxyäthoxy)phenoxy]-2-propanol, 1-/2-Chlor-4-[2-(phenäthyloxy)äthoxy]phenoxy/-3-isopropylamino-2-propanol, 1-/2-Methyl-4-[2-(phenäthyloxy)äthoxy]phenoxy/-3-isopropylamino-2-propanol oder ein pharmazeutisch annehmbares Säureadditionssalz davon.

7. 1-/4-[2-(4-Methoxyphenäthyloxy)äthoxy]phenoxy/-3-isopropylamino-2-propanol, 1-/4-[2-(4-Chlorphenäthyloxy)-äthoxy]phenoxy/-3-isopropylamino-2-propanol, 1-/2-Fluor-4-[2-(4-fluorphenäthyloxy)äthoxy]phenoxy/-3-isopropylamino-2-propanol, 1-/3-Fluor-4-[2-(4-fluorphenäthyloxy)äthoxy]phenoxy/-3-isopropylamino-2-propanol, 1-/4-[2-(2,4-Difluorphenäthyloxy)äthoxy]phenoxy/-3-isopropylamino-2-propanol oder ein pharmazeutisch annehmbares Säureadditionssalz davon.

8. Epoxide der allgemeinen Formel:

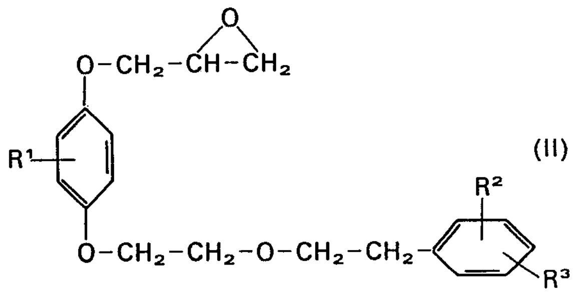

(II)

worin $R^1$ Wasserstoff, Halogen oder niederes Alkyl und $R^2$ und $R^3$ je Wasserstoff, Halogen, niederes Alkyl, niederes Alkoxy oder niederes Alkylthio bedeuten.

9. Phenole der allgemeinen Formel:

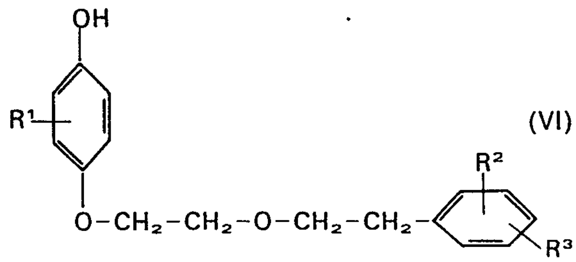

(VI)

worin $R^1$ Wasserstoff, Halogen oder niederes Alkyl und $R^2$ und $R^3$ je Wasserstoff, Halogen, niederes Alkyl, niederes Alkoxy oder niederes Alkylthio bedeuten.

10. Verbindungen der allgemeinen Formel:

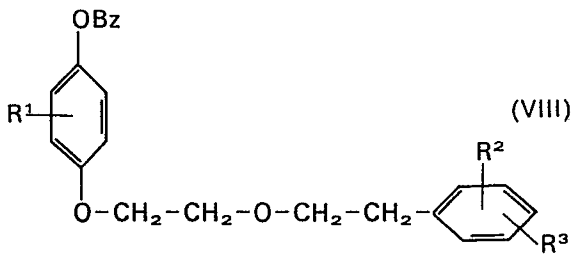

(VIII)

worin $R^1$ Wasserstoff, Halogen oder niederes Alkyl, $R^2$ und $R^3$ je Wasserstoff, Halogen, niederes Alkyl, niederes Alkoxy oder niederes Alkylthio und Bz Benzyl bedeuten.

11. Verbindungen der allgemeinen Formel:

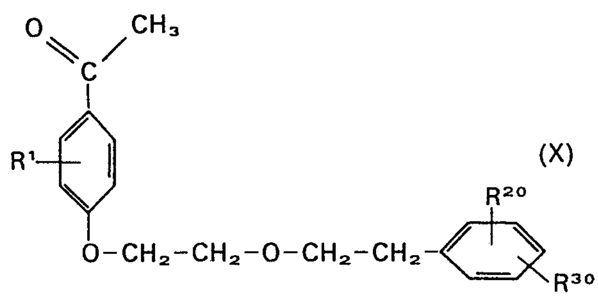

(X)

worin $R^1$ Wasserstoff, Halogen oder niederes Alkyl und $R^{20}$ und $R^{30}$ je Wasserstoff, Halogen, niederes Alkyl oder niederes Alkoxy bedeuten.

12. Substituierte Phenoxy-aminopropanol-Derivate gemäss einem der Ansprüche 1 bis 4, 6 und 7 als pharmazeutische Wirkstoffe.

13. Substituierte Phenoxy-aminopropanol-Derivate gemäss einem der Ansprüche 1 bis 4, 6 und 7 als kardioselektive β-adrenergische Blokkermittel und als antihypertensive Wirkstoffe.

14. 1-/4-[2-(4-Fluorphenäthyloxy)äthoxy]-phenoxy/-3-isopropylamino-2-propanol oder ein pharmazeutisch annehmbares Säureadditionssalz davon als pharmazeutischer Wirkstoff.

15. 1-/4-[2-(4-Fluorphenäthyloxy)äthoxy]-phenoxy/-3-isopropylamino-2-propanol oder ein pharmazeutisch annehmbares Säureadditionssalz davon als kardioselektives β-adrenergisches Blokkermittel oder als antihypertensiver Wirkstoff.

16. Verfahren zur Herstellung von substituierten Phenoxy-aminopropanol-Derivaten der in Anspruch 1 definierten allgemeinen Formel I und von pharmazeutisch annehmbaren Säureadditionssalzen davon, dadurch gekennzeichnet, dass man:

a) ein Epoxid der allgemeinen Formel:

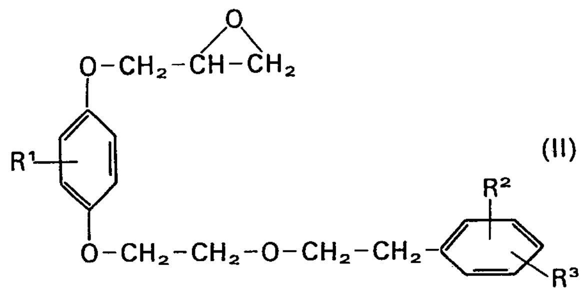

(II)

worin $R^1$, $R^2$ und $R^3$ die in Anspruch 1 angegebene Bedeutung besitzen, mit einem Amin der allgemeinen Formel:

$$H_2N-R \quad (III)$$

worin R die in Anspruch 1 angegebene Bedeutung besitzt, umsetzt, oder

b) ein Alkalimetall-Derivat eines Phenols der allgemeinen Formel:

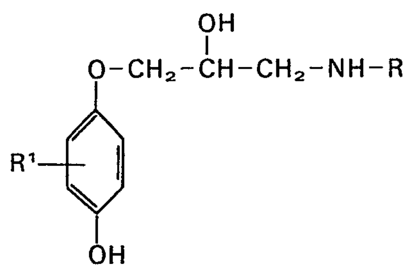

(IV)

worin R und $R^1$ die in Anspruch 1 angegebene Bedeutung besitzen, mit einer Verbindung der allgemeinen Formel:

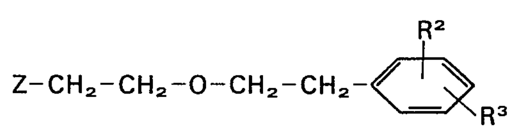

(V)

worin $R^2$ und $R^3$ die in Anspruch 1 angegebene Bedeutung besitzen und Z niederes Alkylsulfonyloxy oder Arylsulfonyloxy bedeutet, umsetzt, und

c) erwünschtenfalls ein erhaltenes Racemat in die optischen Antipoden spaltet, und/oder

d) erwünschtenfalls eine erhaltene Verbindung der allgemeinen Formel I in ein pharmazeutisch annehmbares Säureadditionssalz überführt.

17. Verfahren gemäss Anspruch 16 dadurch gekennzeichnet, dass man ein Epoxid der in Anspruch 16 definierten allgemeinen Formel II, worin $R^1$ in ortho-Stellung zur 2,3-Epoxypropoxygruppe steht, und $R^2$ Wasserstoff, Halogen, niederes Alkyl, niederes Alkoxy oder niederes Alkylthio und $R^3$ Wasserstoff bedeuten, mit einem Amin der in Anspruch 16 definierten allgemeinen Formel III umsetzt oder ein Alkalimetall-Derivat eines Phenols der in Anspruch 16 definierten allgemeinen Formel IV, worin $R^1$ Wasserstoff bedeutet, mit einer Verbindung der in Anspruch 16 definierten allgemeinen Formel V, worin $R^2$ Wasserstoff, Halogen, niederes Alkyl, niederes Alkoxy oder niederes Alkylthio und $R^3$ Wasserstoff bedeuten, umsetzt und erwünschtenfalls ein erhaltenes Racemat in die optischen Antipoden spaltet und/oder erwünschtenfalls eine erhaltene Verbindung der allgemeinen Formel I in ein pharmazeutisch annehmbares Säureadditionssalz überführt.

18. Arzneimittel, enthaltend ein substituiertes Phenoxy-aminopropanol-Derivat gemäss einem der Ansprüche 1 bis 4, 6 und 7.

19. Kardioselektive β-adrenergische Blocker oder Antihypertensiva, enthaltend ein substituiertes Phenoxy-amino-propanol-Derivat gemäss einem der Ansprüche 1 bis 4, 6 und 7.

20. Arzneimittel, enthaltend 1-/4-[2-(4-Fluorphenäthyloxy)äthoxy]phenoxy/-3-isopropylamino-2-propanol oder ein pharmazeutisch annehmbares Säureadditionssalz davon.

21. Kardioselektive β-adrenergische Blocker oder Antihypertensiva, enthaltend 1-/4-[2-(4-Fluorphenäthyloxy)äthoxy]phenoxy/-3-isopropylamino-2-propanol oder ein pharmazeutisch annehmbares Säureadditionssalz davon.

**Patentansprüche für den Vertragsstaat:** AT

1. Verfahren zur Herstellung von substituierten Phenoxyaminopropanol-Derivaten der allgemeinen Formel:

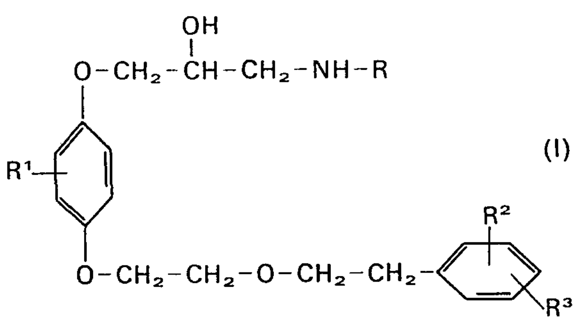

(I)

worin R eine verzweigte, 3 bis 4 Kohlenstoffatome enthaltende Alkylgruppe, $R^1$ Wasserstoff, Halogen oder niederes Alkyl und $R^2$ und $R^3$ je Wasserstoff, Halogen, niederes Alkyl, niederes Alkoxy oder niederes Alkylthio bedeuten, und von pharmazeutisch annehmbaren Säureadditionssalzen davon, dadurch gekennzeichnet, dass man

a) ein Epoxid der allgemeinen Formel:

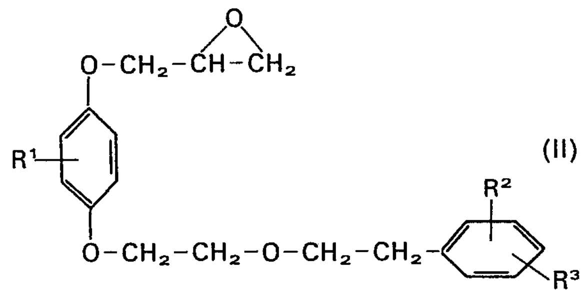

(II)

worin $R^1$, $R^2$ und $R^3$ obige Bedeutung besitzen, mit einem Amin der allgemeinen Formel:

$$H_2N-R \quad (III)$$

worin R obige Bedeutung besitzt, umsetzt, oder

b) ein Alkalimetall-Derivat eines Phenols der allgemeinen Formel:

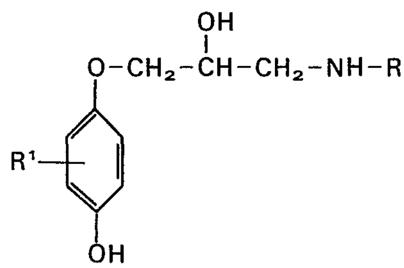

(IV)

worin R und $R^1$ obige Bedeutung besitzen, mit einer Verbindung der allgemeinen Formel:

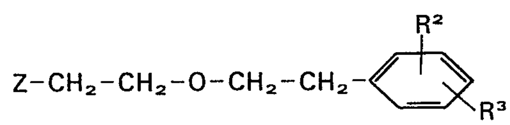

(V)

worin $R^2$ und $R^3$ obige Bedeutung besitzen und Z niederes Alkylsulfonyloxy oder Arylsulfonyloxy bedeutet, umsetzt, und

c) erwünschtenfalls ein erhaltenes Racemat in die optischen Antipoden spaltet, und/oder

d) erwünschtenfalls eine erhaltene Verbindung der allgemeinen Formel I in ein pharmazeutisch annehmbares Säureadditionssalz überführt.

2. Verfahren gemäss Anspruch 1, dadurch gekennzeichnet, dass man ein Epoxid der in Anspruch 1 definierten allgemeinen Formel II, worin $R^1$ in ortho-Stellung zur 2,3-Epoxypropoxygruppe steht, und $R^2$ Wasserstoff, Halogen, niederes Alkyl, niederes Alkoxy oder niederes Alkylthio und $R^3$ Wasserstoff bedeuten, mit einem Amin der in Anspruch 1 definierten allgemeinen Formel III umsetzt oder ein Alkalimetall-Derivat eines Phenols der in Anspruch 1 definierten allgemeinen Formel IV, worin $R^1$ Wasserstoff bedeutet, mit einer Verbindung der in Anspruch 1 definierten allgemeinen Formel V, worin $R^2$ Wasserstoff, Halogen, niederes Alkyl, niederes Alkoxy oder niederes Alkylthio und $R^3$ Wasserstoff bedeuten, umsetzt und erwünschtenfalls ein erhaltenes Racemat in die optischen Antipoden spaltet und/oder erwünschtenfalls eine erhaltene Verbindung der allgemeinen Formel I in ein pharmazeutisch annehmbares Säureadditionssalz überführt.

3. Verfahren gemäss Anspruch 1 oder 2, dadurch gekennzeichnet, dass man eine Verbindung

der in Anspruch 1 definierten allgemeinen Formel I, worin R Isopropyl oder t-Butyl bedeutet, und pharmazeutisch annehmbare Säure-additionssalze davon herstellt.

4. Verfahren gemäss Anspruch 1 oder 2, dadurch gekennzeichnet, dass man 1-Isopropylamino-3-[4-(2-phenäthyloxyäthoxy)phenoxy]-2-propanol oder ein pharmazeutisch annehmbares Säureadditionssalz davon herstellt.

5. Verfahren gemäss Anspruch 1 oder 2, dadurch gekennzeichnet, dass man 1-/4-[2-(4-Fluorphenäthyloxy)äthoxy]phenoxy/-3-isopropylamino-2-propanol oder ein pharmazeutisch annehmbares Säureadditionssalz davon herstellt.

**Claims for the contracting States:**
BE, CH, DE, FR, GB, IT, LI, LU, NL, SE

1. Substituted phenoxy-aminopropanol derivatives of the general formula:

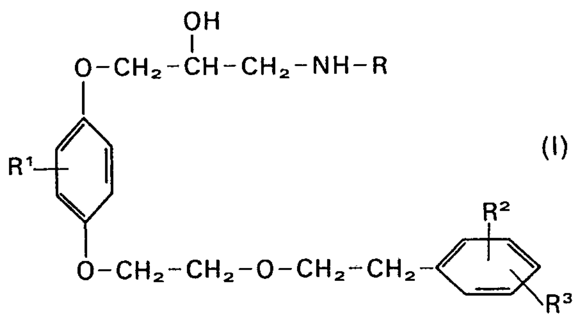

(I)

wherein R represents a branched-chain alkyl group containing 3 to 4 carbon atoms, $R^1$ represents a hydrogen or halogen atom or a lower alkyl group and $R^2$ and $R^3$ each represent a hydrogen or halogen atom or a lower alkyl, lower alkoxy or lower alkylthio group, and pharmaceutically acceptable acid addition salts thereof.

2. Compounds according to claim 1, wherein $R^1$ is present in the ortho-position to the 3-alkylamino-2-hydroxypropoxy group and $R^3$ represents a hydrogen atom, and pharmaceutically acceptable acid addition salts thereof.

3. Compounds according to claim 1 or claim 2, wherein R represents the isopropyl or tert.butyl group, and pharmaceutically acceptable acid addition salts thereof.

4. 1-Isopropylamino-3-[4-(2-phenethyloxyethoxy)phenoxy]-2-propanol or a pharmaceutically acceptable acid addition salt thereof.

5. 1-[4-[2-(4-Fluorophenethyloxy)ethoxy]phenoxy]-3-isopropylamino-2-propanol or a pharmaceutically acceptable acid addition salt thereof.

6. 1-[4-[2-(4-Methylphenethyloxy)ethoxy]phenoxy]-3-isopropylamino-2-propanol,
1-[4-[2-(4-methylthiophenethyloxy)ethoxy]phenoxy]-3-isopropylamino-2-propanol,
1-tert.butylamino-3-[4-(2-phenethyloxyethoxy)phenoxy]-2-propanol,
1-[2-chloro-4-[2-phenethyloxy)ethoxy]phenoxy]-3-isopropylamino-2-propanol,
1-[2-methyl-4-[2-(phenethyloxy)ethoxy]phenoxy]-3-isopropylamino-2-propanol
or a pharmaceutically acceptable acid addition salt thereof.

7. 1-[4-[2-(4-Methoxyphenethyloxy)ethoxy]phenoxy]-3-isopropylamino-2-propanol,
1-[4-[2-(4-chlorophenethyloxy)ethoxy]phenoxy]-3-isopropylamino-2-propanol,
1-[2-fluoro-4-[2-(4-fluorophenethyloxy)ethoxy]phenoxy]-3-isopropylamino-2-propanol,
1-[3-fluoro-4-[2-(4-fluorophenethyloxy)ethoxy]phenoxy]-3-isopropylamino-2-propanol,
1-[4-[2-(2,4-difluorophenethyloxy)ethoxy]phenoxy]-3-isopropylamino-2-propanol
or a pharmaceutically acceptable acid addition salt thereof.

8. Epoxides of the general formula:

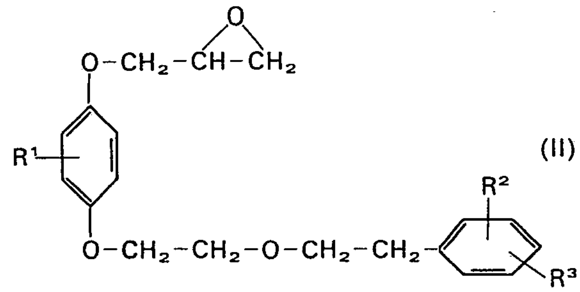

(II)

wherein $R^1$ represents a hydrogen or halogen atom or a lower alkyl group and $R^2$ and $R^3$ each represent a hydrogen or halogen atom or a lower alkyl, lower alkoxy or lower alkylthio group.

9. Phenols of the general formula:

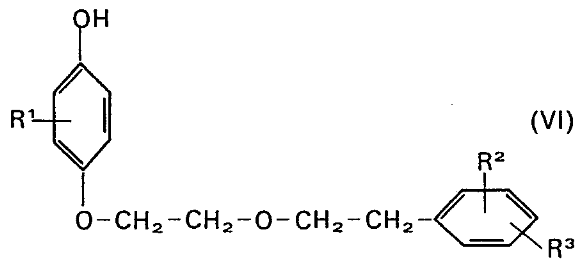

(VI)

wherein $R^1$ represents a hydrogen or halogen atom or a lower alkyl group and $R^2$ and $R^3$ each represent a hydrogen or halogen atom or a lower alkyl, lower alkoxy or lower alkylthio group.

10. Compounds of the general formula:

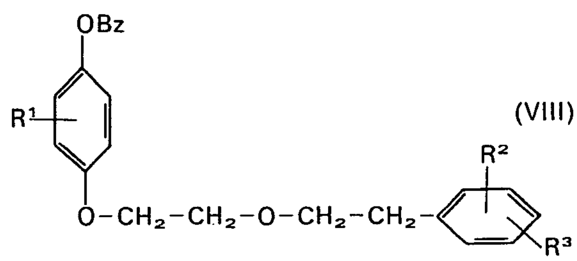

(VIII)

wherein $R^1$ represents a hydrogen or halogen atom or a lower alkyl group, $R^2$ and $R^3$ each represent a hydrogen or halogen atom or a lower alkyl, lower alkoxy or lower alkylthio group and Bz represents a benzyl group.

11. Compounds of the general formula:

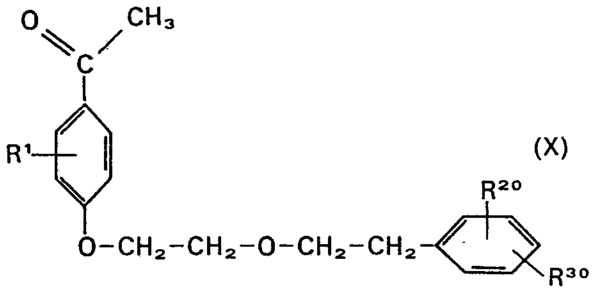

(X)

wherein $R^1$ represents a hydrogen or halogen atom or a lower alkyl group and $R^{20}$ and $R^{30}$ each represent a hydrogen or halogen atom or a lower alkyl or lower alkoxy group.

12. Substituted phenoxy-aminopropanol derivatives according to any one of claims 1 to 4, 6 and 7 as pharmaceutically active substances.

13. Substituted phenoxy-aminopropanol derivatives according to any one of claims 1 to 4, 6 and 7 as cardioselective β-andrenergic blocking or antihypertensive agents.

14. 1-[4-[2-(4-Fluorphenethyloxy)ethoxy]phenoxy]-3-isopropylamino-2-propanol or a pharmaceutically acceptable acid addition salt thereof as pharmaceutically active substance.

15. 1-[4-[2-(4-Fluorphenethyloxy)ethoxy]phenoxy]-3-isopropylamino-2-propanol or a pharmaceutically acceptable acid addition salt thereof as cardioselective β-adrenergic blocking or antihypertensive agent.

16. A process for the manufacture of substituted phenoxyaminopropanol derivatives of formula I given in claim 1 and pharmaceutically acceptable acid addition salts thereof, which process comprises

a) reacting an epoxide of the general formula:

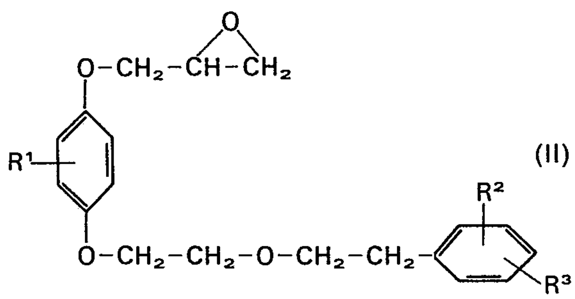

(II)

wherein $R^1$, $R^2$ and $R^3$ have the significance given in claim 1, with an amine of the general formula:

$$H_2N-R \qquad (III)$$

wherein R has the significance given in claim 1, or

b) reacting an alkali metal derivative of a phenol of the general formula:

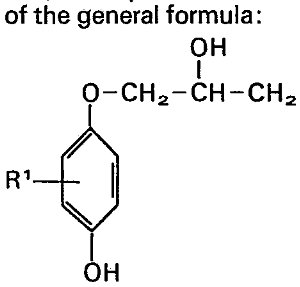

(IV)

wherein R and $R^1$ have the significance given in claim 1, with a compound of the general formula:

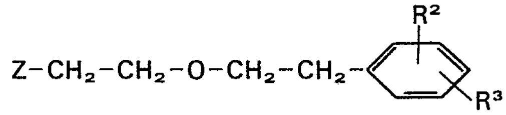

(V)

wherein $R^2$ and $R^3$ have the significance given in claim 1 and Z represents a lower alkylsulphonyloxy or arylsulphonyloxy group, and

c) if desired, resolving a racemate obtained into the optical isomers, and/or

d) if desired, converting a compound obtained of the general formula I into a pharmaceutically acceptable acid addition salt.

17. A process according to claim 16, wherein an epoxide of formula II given in claim 16 in which $R^1$ is present in the ortho-position to the 2,3-epoxypropoxy group, $R^2$ represents a hydrogen or halogen atom or a lower alkyl, lower alkoxy or lower alkylthio group and $R^3$ represents a hydrogen atom is reacted with an amine of formula III given in claim 16 or an alkali metal derivative of a phenol of formula IV given in claim 16 in which $R^1$ represents a hydrogen atom is reacted with a compound of formula V given in claim 16 in which $R^2$ represents a hydrogen or halogen atom or a lower alkyl, lower alkoxy or lower alkylthio group and $R^3$ represents a hydrogen atom, and if desired, a racemate obtained is resolved into the optical isomers, and/or if desired, a compound obtained of the general formula I in converted into a pharmaceutically acceptable acid addition salt.

18. Medicaments containing a substituted phenoxy-aminopropanol derivative according to any one of claims 1 to 4, 6 and 7.

19. Cardioselective β-adrenergic blocking or antihypertensive agents containing a substituted phenoxy-aminopropanol derivative according to any one of claims 1 to 4, 6 and 7.

20. Medicaments containing 1-[4-[2-(4-fluorophenethyloxy)ethoxy]phenoxy]-3-isopropylamino-2-propanol or a pharmaceutically acceptable acid addition salt thereof.

21. Cardioselective β-adrenergic blocking or antihypertensive agents containing 1-[4-[2-(4-fluorophenethyloxy)-ethoxy]phenoxy]-3-isopropylamino-2-propanol or a pharmaceutically acceptable acid addition salt thereof.

**Claims for the contracting State:** AT

1. Process for preparing substituted phenoxy-aminopropanol derivatives of the general formula:

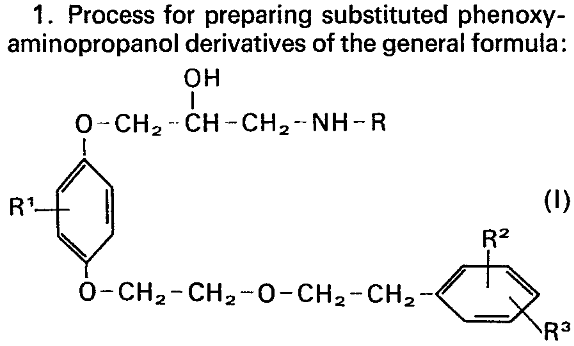

(I)

wherein R represents a branched-chain alkyl group containing 3 to 4 carbon atoms, $R^1$ represents a hydrogen or halogen atom or a lower alkyl group and $R^2$ and $R^3$ each represent a hydrogen or halogen atom or a lower alkyl, lower alkoxy or lower alkylthio group, and pharmaceutically acceptable acid addition salts thereof, which process comprises:

a) reacting an epoxide of the general formula:

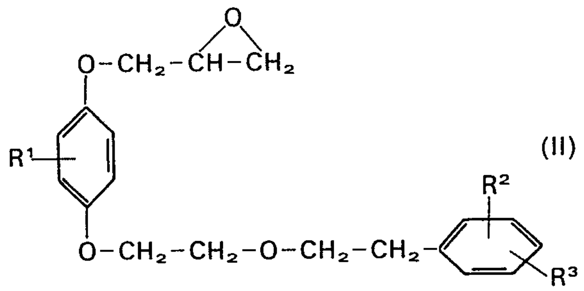

(II)

wherein $R^1$, $R^2$ and $R^3$ have the significance given earlier in this claim, with an amine of the general formula:

$$H_2N-R \qquad (III)$$

wherein R has the significance given earlier in this claim, or

b) reacting an alkali metal derivative of a phenol of the general formula:

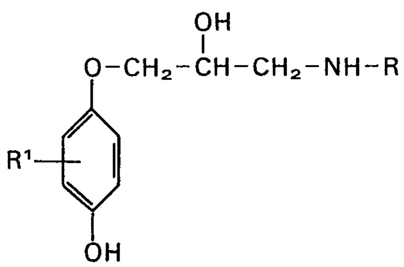

(IV)

wherein R and $R^1$ have the significance given earlier in this claim, with a compound of the general formula:

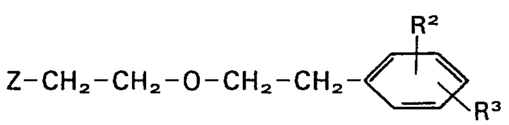

(V)

wherein $R^2$ and $R^3$ have the significance given earlier in this claim and Z represents a lower alkylsulphonyloxy or arylsulphonyloxy group, and

c) if desired, resolving a racemate obtained into the optical isomers, and/or

d) if desired, converting a compound obtained of the general formula I into a pharmaceutically acceptable acid addition salt.

2. A process according to claim 1, wherein an epoxide of formula II given in claim 1 in which $R^1$ is present in the ortho-position to the 2,3-epoxypropoxy group, $R^2$ represents a hydrogen or halogen atom or a lower alkyl, lower alkoxy or lower alkylthio group and $R^3$ represents a hydrogen atom is reacted with an amine of formula III given in claim 1 or an alkali metal derivative of a phenol of formula IV given in claim 1 in which $R^1$ represents a hydrogen atom is reacted with a compound of formula V given in claim 1 in which $R^2$ represents a hydrogen or halogen atom or a lower alkyl, lower alkoxy or lower alkylthio group and $R^3$ represents a hydrogen atom, and if desired, a racemate obtained is resolved into the optical isomers, and/or if desired, a compound obtained of the general formula I is converted into a pharmaceutically acceptable acid addition salt.

3. A process according to claim 1 or 2, wherein R represents the isopropyl or tert.butyl group, and pharmaceutically acceptable acid addition salts thereof.

4. A process as claimed in claim 1 or 2, wherein 1-isopropylamino-3-[4-(2-phenethyloxy-ethoxy)phenoxy]-2-propanol or a pharmaceutically acceptable acid addition salt thereof is prepared.

5. A process as claimed in claim 1 or 2, wherein 1-[4-[2-(4-fluorophenethyloxy)ethoxy]phenoxy]-3-isopropylamino-2-propanol or a pharmaceutically acceptable acid addition salt thereof is prepared.

**Revendications pour les Etats contractants: BE, CH, DE, FR, GB, IT, LI, LU, NL, SE**

1. Dérivés substitués du phénoxyaminopropanol de formule générale:

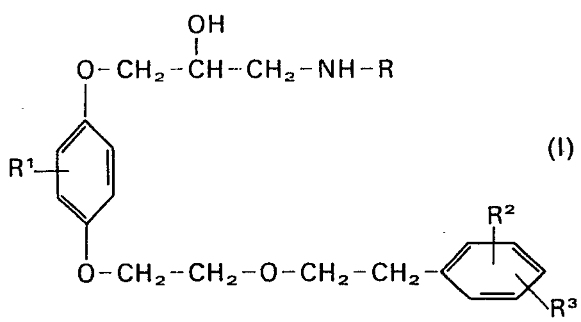

(I)

dans laquelle:

R représente un groupe alkyle ramifié contenant 3 à 4 atomes de carbone,

$R^1$ représente l'hydrogène, un halogène ou un groupe alkyle inférieur, et

$R^2$ et $R^3$ représentent chacun l'hydrogène, un halogène ou un groupe alkyle inférieur, alcoxy inférieur ou alkylthio inférieur,

et leurs sels d'addition d'acides pharmaceutiquement acceptables.

2. Composés selon la revendication 1, caractérisés en ce que $R^1$ est en position ortho du groupe 3-alkylamino-2-hydroxypropoxy et $R^3$ représente l'hydrogène, et leurs sels d'addition d'acides pharmaceutiquement acceptables.

3. Composés selon la revendication 1 ou 2, caractérisés en ce que R représente un groupe isopropyle ou tert.-butyle, et leurs sels d'addition d'acides pharmaceutiquement acceptables.

4. Le 1-isopropylamino-3-[4-(2-phénéthyloxyéthoxy)-phénoxy]-2-propanol ou ses sels d'addition d'acides pharmaceutiquement acceptables.

5. Le1-/4-[2-(4-fluorophénéthyloxy)éthoxy]-phénoxy/-3-isopropylamino-2-propanol ou ses sels d'addition d'acides pharmaceutiquement acceptables.

6. Le 1-/4-[2-(4-méthylphénéthyloxy)éthoxy]-phénoxy/-3-isopropylamino-2-propanol, le 1-/4-[2-(4-méthylthiophénéthyloxy)éthoxy]-phénoxy/-3-isopropylamino-2-propanol, le 1-tert.-butylamino-3-[4-(2-phénéthyloxyéthoxy)-phénoxy]-2-propanol, le 1-/2-chloro-4-[2-(phénéthyloxy)-éthoxy]phénoxy/-3-isopropylamino-2-propanol, le 1-/2-méthyl-4-[2-(phénéthyloxy)éthoxy]phénoxy/-3-isopropylamino-2-propanol ou leurs sels d'addition d'acides pharmaceutiquement acceptables.

7. Le 1-/4-[2-(4-méthoxyphénéthyloxy)éthoxy]phénoxy/-3-isopropylamino-2-propanol, le 1-/4-[2-(4-chlorophénéthyloxy)éthoxy]phénoxy/-3-isopropylamino-2-propanol, le 1-/2-fluoro-4-[2-(4-fluorophénéthyloxy)éthoxy]-phénoxy/-3-isopropylamino-2-propanol, le 1-/3-fluoro-4-[2-(4-fluorophénéthyloxy)éthoxy]-phénoxy/-3-isopropylamino-2-propanol, le 1-/4-[2-(2,4-difluorophénéthyloxy)éthoxy]-phénoxy/-3-isopropylamino-2-propanol ou leurs sels d'addition d'acides pharmaceutiquement acceptables.

8. Epoxydes de formule générale:

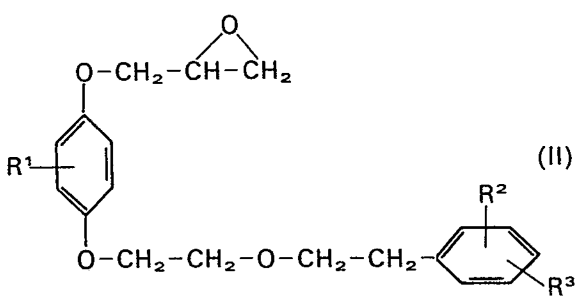

(II)

dans laquelle:
$R^1$ représente l'hydrogène, un halogène ou un groupe alkyle inférieur, et
$R^2$ et $R^3$ représentent chacun l'hydrogène, un halogène ou un groupe alkyle inférieur, alcoxy inférieur ou alkylthio inférieur.

9. Phénols de formule générale:

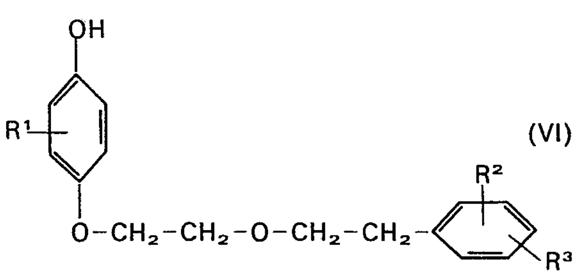

(VI)

dans laquelle:
$R^1$ représente l'hydrogène, un halogène ou un groupe alkyle inférieur, et
$R^2$ et $R^3$ représentent chacun l'hydrogène, un halogène ou un groupe alkyle inférieur, alcoxy inférieur ou alkylthio inférieur.

10. Composés de formule générale:

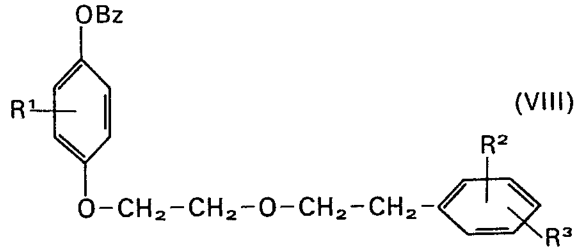

(VIII)

dans laquelle:
$R^1$ représente l'hydrogène, un halogène ou un groupe alkyle inférieur,
$R^2$ et $R^3$ représentent chacun l'hydrogène, un halogène ou un groupe alkyle inférieur, alcoxy inférieur ou alkylthio inférieur, et
Bz représente le groupe benzyle.

11. Composés de formule générale:

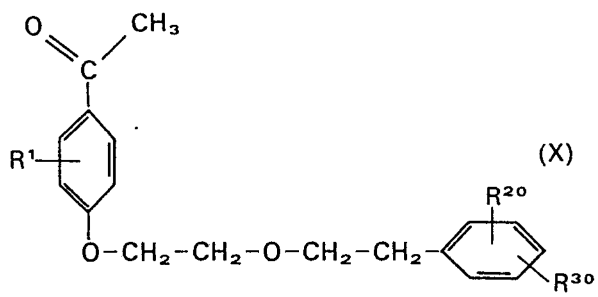

(X)

dans laquelle:
$R^1$ représente l'hydrogène, un halogène ou un groupe alkyle inférieur, et
$R^{20}$ et $R^{30}$ représentent chacun l'hydrogène, un halogène ou un groupe alkyle inférieur ou alcoxy inférieur.

12. Dérivés substitués du phénoxyaminopropanol selon l'une des revendications 1 à 4, 6 et 7, en tant que substances actives pharmaceutiques.

13. Dérivés substitués du phénoxyaminopropanol selon l'une des revendications 1 à 4 et 6 et 7, en tant qu'agents bloquants β-adrénergiques cardiosélectifs et en tant que substances actives antihypertensives.

14. Le 1-/4-[2-(4-fluorophénéthyloxy)éthoxy]-phénoxy/-3-isopropylamino-2-propanol ou un sel d'addition d'acides pharmaceutiquement acceptables de ce composé en tant que substance active pharmaceutique.

15. Le 1-/4-[2-(4-fluorophénéthyloxy)éthoxy]-phénoxy/-3-isopropylamino-2-propanol ou un sel d'addition d'acides pharmaceutiquement acceptables de ce composé en tant qu'agent bloquant β-adrénergique cardiosélectif ou en tant que substance active antihypertensive.

16. Procédé de préparation des dérivés substitués du phénoxyaminopropanol de formule générale I définie dans la revendication 1 et de leurs sels d'addition d'acides pharmaceutiquement acceptables, caractérisé en ce que:
a) on fait réagir un époxyde de formule générale:

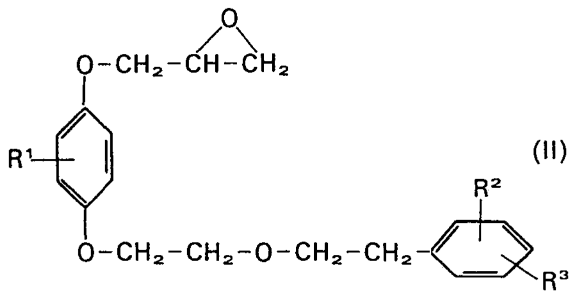

(II)

dans laquelle $R^1$, $R^2$ et $R^3$ ont les significations indiquées dans la revendication 1, avec une amine de formule générale:

$$H_2N-R \quad (III)$$

dans laquelle R a la signification indiquée dans la revendication 1, ou bien:

b) on fait réagir un dérivé de métal alcalin d'un phénol de formule générale:

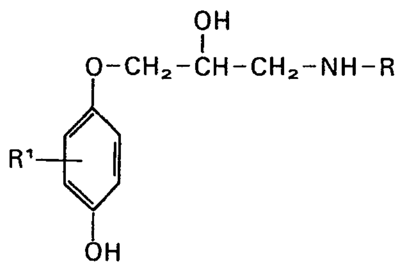

(IV)

dans laquelle R et $R^1$ ont les significations indiquées dans la revendication 1, avec un composé de formule générale:

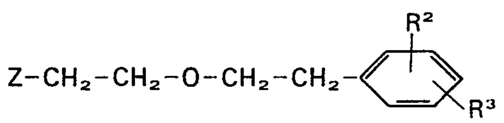

(V)

dans laquelle $R^2$ et $R^3$ ont les significations indiquées dans la revendication 1 et Z représente un groupe alkylsulfonyloxy inférieur ou arylsulfonyloxy, et

c) si on le désire, on résoud un racémate obtenu en les antipodes optiques, et/ou

d) si on le désire, on convertit un composé obtenu répondant à la formule générale I en un sel d'addition d'acides pharmaceutiquement acceptable.

17. Procédé selon la revendication 16, caractérisé en ce que l'on fait réagir un époxyde de formule générale II définie dans la revendication 16 dans laquelle $R^1$ est en position ortho du groupe 2,3-époxypropoxy, et $R^2$ représente l'hydrogène, un halogène ou un groupe alkyle inférieur, alcoxy inférieur ou alkylthio inférieur, et $R^3$ représente l'hydrogène, avec une amine de formule générale III définie dans la revendication 16, ou bien on fait réagir un dérivé de métal alcalin d'un phénol de formule générale IV définie dans la revendication 16 dans laquelle $R^1$ représente l'hydrogène, avec un composé de formule générale V définie dans la revendication 16 dans laquelle $R^2$ représente l'hydrogène, un halogène ou un groupe alkyle inférieur, alcoxy inférieur ou alkylthio inférieur, et $R^3$ représente l'hydrogène et, si on le désire, on résout un racémate obtenu en les antipodes optiques et/ou, si on le désire, on convertit un composé obtenu répondant à la formule générale I en un sel d'addition d'acides pharmaceutiquement acceptable.

18. Médicaments contenant un dérivé substitué du phénoxyaminopropanol selon l'une des revendications 1 à 4, 6 et 7.

19. Agents bloquants β-adrénergiques cardiosélectifs ou agents antihypertensifs contenant un dérivé substitué du phénoxyaminopropanol selon l'une des revendications 1 à 4, 6 et 7.

20. Médicaments contenant du 1-/4-[2-(4-fluorophénéthyloxy)éthoxy]phénoxy/-3-isopropylamino-2-propanol ou un sel d'addition d'acides pharmaceutiquement acceptable de ce composé.

21. Agents bloquants β-adrénergiques cardiosélectifs ou agents antihypertensifs contenant du 1-/4-[2-(4-fluorophénéthyloxy)éthoxy]phénoxy/-3-isopropylamino-2-propanol ou un sel d'addition d'acides pharmaceutiquement acceptable de ce composé.

**Revendications pour l'Etat contractant:** AT

1. Procédé de préparation de dérivés substitués du phénoxyaminopropanol de formule générale:

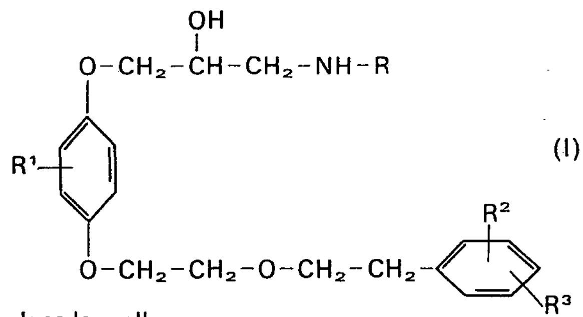

(I)

dans laquelle:

R représente un groupe alkyle ramifié contenant 3 à 4 atomes de carbone,

$R^1$ représente l'hydrogène, un halogène ou un groupe alkyle inférieur, et

$R^2$ et $R^3$ représentent chacun l'hydrogène, un halogène ou un groupe alkyle inférieur, alcoxy inférieur ou alkylthio inférieur,

et de leurs sels d'addition d'acides pharmaceutiquement acceptables, caractérisé en ce que:

a) on fait réagir un époxyde de formule générale:

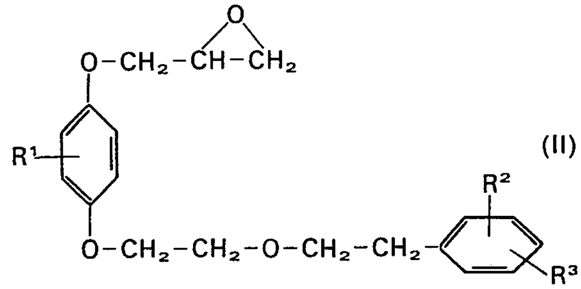

(II)

dans laquelle $R^1$, $R^2$ et $R^3$ ont les significations indiquées ci-dessus, avec une amine de formule générale:

$H_2N-R$ (III)

dans laquelle R a la signification indiquée ci-dessus, ou bien

b) on fait réagir un dérivé de métal alcalin d'un phénol de formule générale:

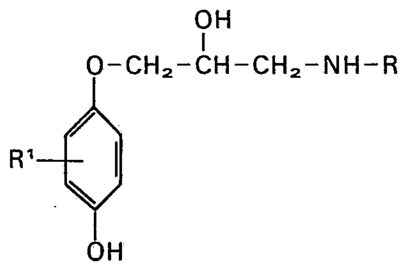

(IV)

dans laquelle R et $R^1$ ont les significations indiquées ci-dessus, avec un composé de formule générale:

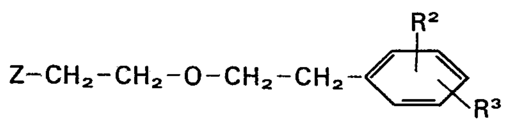

(V)

dans laquelle $R^2$ et $R^3$ ont les significations indiquées ci-dessus, et Z représente un groupe alkylsulfonyloxy inférieur ou arylsulfonyloxy, et

c) si on le désire, on résoud un racémate obtenu en les antipodes optiques, et/ou

d) si on le désire, on convertit un composé obtenu répondant à la formule générale I en un sel d'addition d'acides pharmaceutiquement acceptable.

2. Procédé selon la revendication 1, caractérisé en ce que l'on fait réagir un époxyde de formule générale II définie dans la revendication 1, dans laquelle $R^1$ est en position ortho du groupe 2,3-époxypropoxy et $R^2$ représente l'hydrogène, un halogène ou un groupe alkyle inférieur, alcoxy inférieur ou alkylthio inférieur, et $R^3$ représente l'hydrogène, avec une amine de formule générale III définie dans la revendication 1, ou bien on fait réagir un dérivé de métal alcalin d'un phénol de formule générale IV définie dans la revendication 1, dans laquelle $R^1$ représente l'hydrogène, avec un composé de formule générale V définie dans la revendication 1, dans laquelle $R^2$ représente l'hydrogène, un halogène ou un groupe alkyle inférieur, alcoxy inférieur ou alkylthio inférieur et, si on le désire, on résoud un racémate obtenu en les antipodes optiques et/ou, si on le désire, on convertit un composé obtenu répondant à la formule générale I en un sel d'addition d'acides pharmaceutiquement acceptable.

3. Procédé selon la revendication 1 ou 2, caractérisé en ce que l'on prépare un composé de formule générale I définie dans la revendication 1, dans laquelle R représente un groupe isopropyle ou tert.-butyle, et ses sels d'addition d'acides pharmaceutiquement acceptables.

4. Procédé selon la revendication 1 ou 2, caractérisé en ce que l'on prépare le 1-isopropylamino-3-[4-(2-phénéthyloxyéthoxy)-phénoxy]-2-propanol ou un sel d'addition d'acides pharmaceutiquement acceptable de ce composé.

5. Procédé selon la revendication 1 ou 2, caractérisé en ce que l'on prépare le 1-/4-[2-(4-fluorophénéthyloxy)éthoxy]phénoxy/-3-isopropylamino-2-propylamino-2-propanol ou un sel d'addition d'acides pharmaceutiquement acceptable de ce composé.